(19) Europäisches Patentamt
European Patent Office
Office européen des brevets

(11) EP 1 466 891 A1

(12) **EUROPEAN PATENT APPLICATION**
published in accordance with Art. 158(3) EPC

(43) Date of publication:
**13.10.2004 Bulletin 2004/42**

(21) Application number: **02793420.7**

(22) Date of filing: **26.12.2002**

(51) Int Cl.[7]: **C07C 235/38**, C07C 255/60, C07C 311/29, C07C 317/32, C07C 323/41, C07D 209/48, C07D 209/76, C07D 261/14, C07D 263/32, C07D 271/10, C07D 277/28, C07D 277/42, C07D 277/38, C07D 295/22, C07D 307/91, C07D 311/14, C07D 333/34 // (A61K31/198, 31:343, 31:352, 31:381, 31:40, 31:403, 31:4035, 31:42)

(86) International application number:
**PCT/JP2002/013692**

(87) International publication number:
**WO 2003/055847 (10.07.2003 Gazette 2003/28)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **27.12.2001 JP 2001396525**

(71) Applicant: **TAISHO PHARMACEUTICAL CO., LTD**
**Tokyo 170-8633 (JP)**

(72) Inventors:
- **SAITO, Shuji,**
**c/o TAISHO PHARMACEUTICAL CO., LTD.**
**Toshima-ku, Tokyo 170-8633 (JP)**
- **SUGA, Yoichiro,**
**c/o TAISHO PHARMACEUTICAL CO. LTD.**
**Toshima-ku, Tokyo 170-8633 (JP)**
- **SATO, Masakazu,**
**c/o TAISHO PHARMACEUTICAL CO. LTD.**
**Toshima-ku, Tokyo 170-8633 (JP)**
- **SHIBUYA, Masabumi**
**Kawaguchi-shi, Saitama 333-0866 (JP)**

(74) Representative: **HOFFMANN EITLE**
**Patent- und Rechtsanwälte**
**Arabellastrasse 4**
**81925 München (DE)**

(54) **CARBOXYLIC ACID DERIVATIVE**

(57) The following compounds useful as VEGF receptor antagonists and having excellent physical properties are provided.

A carboxylic acid derivative represented by formula:

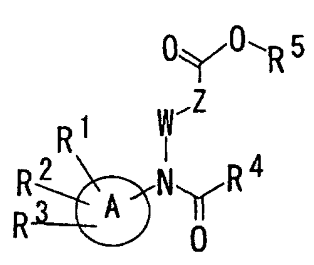

wherein ring A represents a benzene ring, a naphthalene ring, or the like; W represents a $C_{1-5}$ alkylene group; Z represents a single bond or a phenylene group; $R^1$ and $R^2$ are the same or different and each represents a hydrogen atom, a halogen atom, a $C_{1-5}$ alkyl group or a $C_{1-10}$ alkoxy group; $R^3$ represents a hydrogen atom, a halogen atom, a $C_{1-12}$ alkyl group, a $C_{2-5}$ alkynyl group, a trifluoromethyl group, an acetynyl group, a cyano group, a nitro group, $-CH_2-R^6$, $-Y-R^{11}$, or the like; $R^4$ represents a group represented by formula:

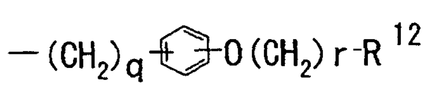 ;

EP 1 466 891 A1

and
$R^5$ represents a hydrogen atom or a $C_{1-5}$ alkyl group, or
a pharmaceutical acceptable salt of the derivative.

## Description

### Technical Field

The present invention relates to a novel carboxylic acid derivative having a vascular endothelial growth factor (hereinafter abbreviated as "VEGF") receptor antagonistic action or a pharmaceutically acceptable salt of the derivative.

### Background Art

VEGF (vascular endothelial growth factor) is a growth factor exhibiting extremely high specificity to vascular endothelial cells. VEGF and the receptors thereof play main roles in physiologic arterialization such as development or placentation. As VEGF receptors, Flt-1 (fms-like tyrosine kinase) and KDR (Kinase inert domain containing receptor) have been reported (Advances in Cancer Research, vol. 67 , pp. 281-316, 1995). It is suggested that VEGF and the receptors thereof play main roles not only in physiologic arterialization but also in pathologic arterialization observed in diseases such as diabetic retinopathy, chronic rheumatism, and solid tumors (Advances in Cancer Research, vol. 67, pp. 281-316, 1995) , and are deeply involved in progress of these diseases. In addition, it is known that VEGF and the receptors thereof are involved not only in arterialization but also in vascular hyperpermeability. It is suggested that vascular hyperpermeability due to VEGF is involved in pathologic symptoms such as carcinomatous ascites retention or cerebral edema upon ischemia reperfusion injury (J. Clin. Invest., vol. 104, pp.1613-1620, 1999). Therefore, it is believed that substances which inhibit binding between VEGF and the receptors thereof are considered to be useful in treatment of various diseases in which pathologic arterialization due to VEGF is involved, and amelioration of pathologic symptoms in which vascular hyperpermeabilitgy due to VEGF is involved. Examples of low-molecular compounds having the VEGF receptor antagonistic action include aminobenzoic acid derivatives described in JP-A-12-72653 (WO01/02344), such as 5-amino-2-(4-carboxyphenoxy)-N-[3-{4-(1-octadecyloxy)phenyl}propionyl]benzoic acid.

However, the solubility in water of the above-described aminobenzoic acid derivatives is not entirely sufficient.

An object of the present invention is to provide compounds useful as a VEGF receptor antagonist for treating diseases, in which arterialization induced by VEGF is involved, and for ameliorating pathologic symptoms induced by VEGF, and having excellent physical properties.

### Disclosure of the Invention

As a result of intensive investigations to solve the problems, the inventors have found that a carboxylic acid derivative represented by the following formula (1) and a pharmaceutically acceptable salt of the derivative have a VEGF receptor antagonistic action and an excellent solubility in water, thus having completed the invention based on the finding.

The present invention is a carboxylic acid derivative represented by formula (1):

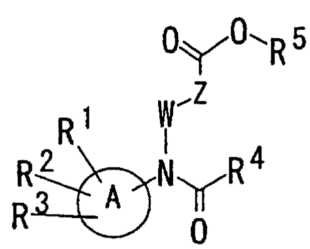

(1)

wherein ring A represents a benzene ring, a naphthalene ring or a hetero ring containing 1 to 4 hetero atoms arbitrarily selected from among a nitrogen atom, an oxygen atom and a sulfur atom,

W represents a $C_{1-5}$ alkylene group,

Z represents a single bond or a phenylene group,

$R^1$ and $R^2$ are the same or different and each represents a hydrogen atom, a halogen atom, a $C_{1-5}$ alkyl group or a $C_{1-10}$ alkoxy group,

$R^3$ represents a hydrogen atom, a halogen atom, a $c_{1-12}$ alkyl group, a $C_{2-5}$ alkynyl group, a trifuloromethyl group, an acetynyl group, a cyano group, a nitro group, a group represented by $-CH_2-R^6$ [wherein $R^6$ represents a $C_{1-5}$ alkyl thio

group, a group represented by formula:

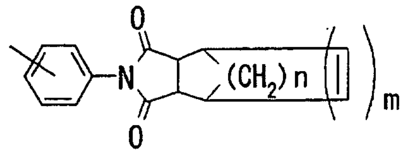

(wherein m represents 0 or 1, and n represents an integer of from 0 to 3)], a group represented by formula:

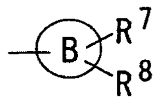

[wherein ring B represents a monocyclic hetero ring containing 1 to 3 hetero atoms arbitrarily selected from among a nitrogen atom, an oxygen atom and a sulfur atom, or a benzene ring, $R^7$ represents a hydrogen atom or a $C_{1-5}$ alkyl group, $R^8$ represents a hydrogen atom, a $C_{1-5}$ alkyl group or a group represented by formula:

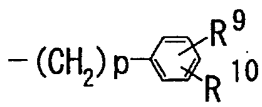

(wherein $R^9$ and $R^{10}$ are the same or different and each represents a hydrogen atom, a halogen atom, a $C_{1-5}$ alkyl group or a $C_{1-5}$ alkoxy group, and p represents an integer of from 0 to 8)], or a group represented by $-Y-R^{11}$ (wherein Y represents a group represented by -CO-, -O-, -S- or $-SO_2-$, and $R^{11}$ represents a $C_{1-10}$ alkyl group, a methyl group substituted by 1 to 3 fluorine atoms, a phenyl group, a phenyl group substituted by a $C_{1-5}$ alkyl group, a phenyl group substituted by a $C_{1-5}$ alkoxy group, a $C_{2-8}$ dialkylamino group or a cyclic amino group)],
$R^4$ represents a group represented by formula:

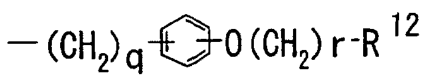

(wherein $R^{12}$ represents a hydrogen atom or a phenyloxy group substituted by a $C_{1-5}$ alkoxy group, q represents an integer of from 1 to 5, and r represents an integer of from 10 to 24), and
$R^5$ represents a hydrogen atom or a $C_{1-5}$ alkyl group, or a pharmaceutically acceptable salt of the derivative.

The terms used in the invention are defined below.

In the present invention, the term "$C_{x-y}$" means that a group following the term has x to y carbon atoms.

The hetero ring containing 1 to 4 hetero atoms arbitrarily selected from among a nitrogen atom, an oxygen atom and a sulfur atom as defined by A is a monocyclic or condensed-ring hetero ring containing 1 to 4 hetero atoms arbitrarily selected from among a nitrogen atom, an oxygen atom and a sulfur atom, and is exemplified by a furan ring, a thiophene ring, a pyrrole ring, an oxazole ring, an isoxazole ring, a thiazole ring, an isothiazole ring, an imidazole ring, a pyrazole ring, an oxadiazole ring, a thiadiazole ring, a tetrazole ring, apyridine ring, a pyridazine ring, a pyrimidine ring, a pyrazine ring, a furanofuran ring, a thienofuran ring, an imidazothiazole ring, a benzofuran ring, a benzothiophene ring, an indole ring, an indolizine ring, a benzoxazole ring, a benzoisoxazole ring, a benzothiazole ring, a benzoisothiazole ring, a benzimidazole ring, a benzpyrazole ring, a benzotriazole ring, a purine ring, a phthalimide ring, a quinoline ring, an isoquinoline ring, a quinazoline ring, a quinoxaline ring, a coumarin ring, an isocoumarin ring, a dibenzofuran ring, a naphthofuran ring, a dibenzothiophene ring, a naphthothiophene ring, an acridine ring, a phenanthridine ring and a phenothiazine ring. Of these, a thiophene ring, a thiazole ring, an isoxazole ring, a benzothiazole ring, a phthalimide ring, a coumarin ring or a dibenzofuran ring is preferred.

Examples of the monocyclic hetero ring containing 1 to 3 hetero atoms arbitrarily selected from among a nitrogen atom, an oxygen atom and a sulfur atom as defined by B include a furan ring, a thiophene ring, a pyrrole ring, an oxazole ring, an isoxazole ring, a thiazole ring, an isothiazole ring, an imidazole ring, a pyrazole ring, an oxadiazole

ring, a thiadiazole ring and a tetrazole ring, with an oxazole ring or an oxadiazole ring being preferred.

The term "$C_{1-5}$ alkylene group" means a straight-chain or branched-chain alkylene group having 1 to 5 carbon atoms, and examples thereof include a methylene group, a methylmethylene group, an ethylene group, a trimethylene group, a methylethylene group, a tetramethylene group, an ethylethylene group, a dimethylethylene group and a pentamethylene group.

The terms "$C_{1-5}$ alkyl group", "$C_{1-10}$ alkyl group" and "$C_{1-12}$ alkyl group" mean, respectively, a straight-chain or branched-chain alkyl group having 1 to 5 carbon atoms, a straight-chain alkyl group having 1 to 10 carbon atoms, and a straight-chain or branched-chain alkyl group having 1 to 12 carbon atoms, and examples thereof include a methyl group, an ethyl group, a n-propyl group, an isopropyl group, a n-butyl group, an isobutyl group, a t-butyl group, a n-pentyl group, an isopentyl group, a n-hexyl group, an isohexyl group, a n-octyl group, and a n-decyl group.

The halogen atoms are a fluorine atom, a chlorine atom, a bromine atom and an iodine atom.

The term "$C_{1-5}$ alkylthio group" means a straight-chain or branched-chain alkylthio group having 1 to 5 carbon atoms, and examples thereof include a methylthio group, an ethylthio group, a n-propylthio group, an isopropylthio group, a n-butylthio group, an isobutylthio group, a t-butylthio group, and a n-pentylthio group.

The term "$C_{1-5}$ alkoxy group" means a straight-chain or branched-chain alkoxy group having 1 to 5 carbon atoms, and examples thereof include a methoxy group, an ethoxy group, a n-propoxy group, an isopropoxy group, a n-butoxy group, an isobutyloxy group, a t-butoxy group, and a n-pentyloxy group. The term "$C_{1-10}$ alkoxy group" means a straight-chain or branched-chain alkoxy group having 1 to 10 carbon atoms, and examples thereof include the above-mentioned ones and, in addition, a n-hexyloxy group, a n-heptyloxy group and a n-decyloxy group.

The term "phenyl group substituted by a $C_{1-5}$ alkyl group" means a phenyl group substituted by a straight-chain or branched-chain alkyl group having 1 to 5 carbon atoms, and examples thereof include a 2-methylphenyl group, a 3-ethylphenyl group, a 2-n-propylphenyl group, a 4-isopropylphenyl group, a 2-n-butylphenyl group, a 2-isobutylphenyl group, a 4-t-butylphenyl group, and a 3-n-pentylphenyl group.

The term "phenyl group substi tuted by a $C_{1-5}$ alkoxy group" means a phenyl group substituted by a straight-chain or branched-chain alkoxy group having 1 to 5 carbon atoms, and examples thereof include a 2-methoxyphenyl group, a 4-ethoxyphenyl group, a 3-n-propoxyphenyl group, a 3-isopropoxyphenyl group, a 3-n-butoxyphenyl group, a 4-isobutyloxyphenyl group, a 4-t-butoxyphenyl group, and a 2-n-pentyloxyphenyl group.

The term "phenyloxy group substituted by a $C_{1-5}$ alkoxy group" means a phenoxy group substituted by a straight-chain or branched-chain alkoxy group having 1 to 5 carbon atoms, and examples thereof include a 3-methoxyphenoxy group, a 4-ethoxyphenoxy group, a 4-n-propoxyphenoxy group, a 3-isopropoxyphenoxy group, a 2-n-butoxyphenoxy group, a 4-isobutyloxyphenoxy group, a 3-t-butoxyphenoxy group, and a 4-n-pentyloxyphenoxy group.

The methyl group substituted by 1 to 3 fluorine atoms is a fluoromethyl group, a difluoromethyl group or a trifluoromethyl group.

The $C_{2-10}$ dialkylamino group is an amino group substituted by two straight-chain or branched-chain alkyl groups having 1 to 5 carbon atoms, which are the same or different, and examples thereof include a dimethylamino group, an N-ethyl-N-methylamino group, a diethylamino group, an N-ethyl-N-isopropylamino group, a dipropylamino group, a diisopropylamino group, a dibutylamino group, a diisobutylamino group and a dipentylamino group.

Examples of the cyclic amino group include an aziridino group, an azetidino group, a pyrrolidino group, a piperidino group, a quinuclidino group and a morpholino group.

Also, in the present invention; examples of the pharmacologically acceptable salt include salts with a mineral acid such as sulfuric acid, hydrochloric acid or phosphoric acid, salts with an organic acid such as acetic acid, oxalic acid, lactic acid, tartaric acid, fumaric acid, maleic acid, methanesulfonic acid or benzenesulfonic acid, salts with an amine such as trimethylamine or methylamine, and salts with a metal ion such as sodium ion, potassium ion or calcium ion.

Some of the compounds according to the present invention exhibit crystal polymorphism. The present invention includes any crystal forms thereof.

The compounds (1) of the present invention can be produced, for example, according to the process described below.

1) The compounds (1) of the present invention wherein $R^5$ represents a $C_{1-5}$ alkyl group, which are referred to as compounds (1a), can be produced according to the process shown by the following reaction formulae:

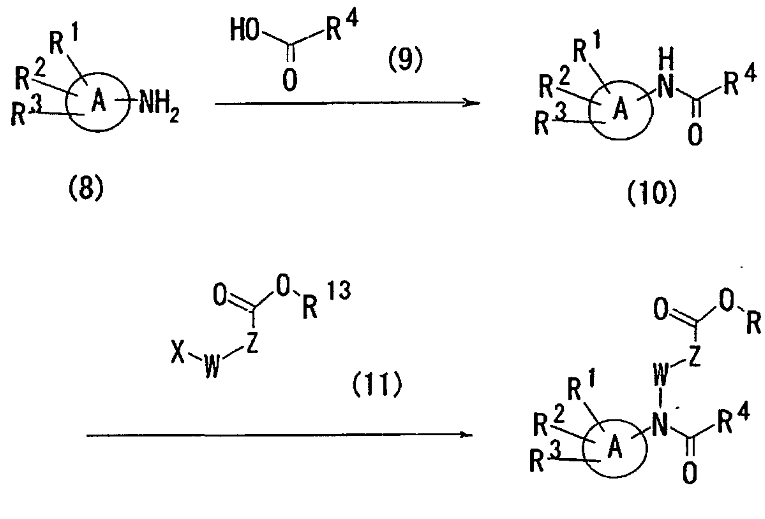

(8) (9) (10)

(11)

(1a)

In the above formulae, A, W, Z, $R^1$, $R^2$, $R^3$ and $R^4$ are the same as defined hereinbefore, $R^{13}$ represents a $C_{1-5}$ alkyl group, and X represents an eliminating group such as a halogen atom, a methanesulfonyloxy group, a trifluoromethanesulfonyloxy group or a p-toluenesulfonyloxy group.

The compound (1a) of the present invention can be produced using an amino compound (8) as a starting material. That is, an amide compound (10) can be produced by condensing the amino compound (8) with a carboxylic acid compound (9).

In this condensation reaction, it is preferred to use a condensing agent. Examples of the condensing agent include a thionyl halide such as thionyl chloride or thionyl bromide, a phosphorus halide such as phosphorus trichloride, phosphorus tribromide or phosphorus pentachloride, a hydrogen halide such as hydrogen chloride, hydrogen bromide or hydrogen iodide , alkyl halocarbonate such as methyl chlorocarbonate, ethyl chlorocarbonate or ethyl bromocarbonate, a carbodiimide compound such as dicyclohexylcarbodiimide or 1-ethyl-3-(3-dimethylamino)propylcarbodiimide, a sulfonyl chloride compound such as methanesulfonyl chloride, a phosphorus compound such as diphenyl phosphate or diphenylphosphoryl chloride, triphenylphosphine-diethyl azadicarboxylate and N,N'-carbodiimidazole.

In the case of using a thionyl halide, a phosphorus halide, an alkyl halocarbonate or the like, it is preferred to conduct the reaction in the presence of a base, and examples of the base include an alkali metal hydroxide such as lithium hydroxide, sodium hydroxide or potassium hydroxide, an alkali metal carbonate such as lithium carbonate, sodium carbonate or potassium carbonate, an alkali metal hydrogen carbonate such as sodium hydrogen carbonate or potassium hydrogen carbonate, an alkali metal hydride such as sodium hydride or potassium hydride, an alkali metal such as metallic sodium or metallic potassium, an alkali metal amide such as sodium amide, ammonia, aqueous ammonia, an organic base such as triethylamine, diisopropylethylamine, tri-n-butylamine, 1,5-diazabicyclo[4.3.0]-5-nonene, 1,8-diazabicyclo[5.4.0]-7-undecene, pyridine or N,N-dimethylaminopyridine, an alkali metal alkoxide such as sodium methoxide, sodium ethoxide or potassium t-butoxide, and an organometallic compound such as methyllithium, n-butyllithium, s-butyllithium, t-butyllithium, lithium N,N-diisopropylamide or sodium bis(trimethylsilyl)amide.

This reaction can be conducted in the absence of or in a solvent. Examples of the solvent to be used include an alcohol such as methanol, ethanol, n-propanol, isopropanol, n-butanol, t-butanol, 2-methoxyethanol or ethylene glycol, an ether such as dioxane, tetrahydrofuran, dimethoxyethane, isopropyl ether or diethyl ether, an nitrile such as acetonitrile, a ketone such as acetone or methyl ethyl ketone, an aliphatic hydrocarbon such as petroleum ether, n-hexane or cyclohexane, an aromatic hydrocarbon such as benzene, toluene, xylene or chlorobenzene, an ester such as ethyl acetate or ethyl propionate, an alkyl halide such as dichloromethane, chloroform, tetrachlorocarbon or 1,2-dichloroethane, pyridine, N,N-dimethylformamide, dimethylsulfoxide, and water.

In this reaction, kinds of the solvent and the reagent and amounts thereof are preferably properly selected depending upon the substrates and reaction conditions employed for the reaction.

The compound (1a) of the present invention can be produced by alkylating the nitrogen atom of the amido group of the amide compound (10) by using an ester compound (11).

This reaction is preferably conducted in the presence of a base, and examples of the base include an alkali metal hydroxide such as lithium hydroxide, sodium hydroxide or potassium hydroxide, an alkali metal carbonate such as lithium carbonate, sodium carbonate or potassium carbonate, an alkali metal hydrogencarbonate such as sodium

hydrogencarbonate or potassium hydrogencarbonate, an alkali metal hydride such as sodium hydride or potassium hydride, an alkali metal such as metallic sodium or metallic potassium, an alkali metal amide such as sodium amide, ammonia, aqueous ammonia, an organic base such as triethylamine, diisopropylethylamine, tri-n-butylamine, 1,5-diazabicyclo[4.3.0]-5-nonene, 1,8-diazabicyclo[5.4.0]-7-undecene, pyridine or N,N-dimethylaminopyridine, an alkali metal alkoxide such as sodium methoxide, sodium ethoxide or potassium t-butoxide, and an organometallic compound such as methyllithium, n-butyllithium, s-butyllithium, t-butyllithium, lithium N,N-diisopropylamide or sodium bis(trimethylsilyl)amide.

This reaction can be conducted in the absence of or in a solvent. Examples of the solvent to be used include an alcohol such as methanol, ethanol, n-propanol, isopropanol, n-butanol, t-butanol, 2-methoxyethanol or ethylene glycol, an ether such as dioxane, tetrahydrofuran, dimethoxyethane, isopropyl ether or diethyl ether, a nitrile such as acetonitrile, a ketone such as acetone or methyl ethyl ketone, an aliphatic hydrocarbon such as petroleum ether, n-hexane or cyclohexane, an aromatic hydrocarbon such as benzene, toluene, xylene or chlorobenzene, an ester such as ethyl acetate or ethyl propionate, an alkyl halide such as dichloromethane, chloroform, tetrachlorocarbon or 1,2-dichloroethane, pyridine, N,N-dimethylformamide, dimethylsulfoxide, and water.

In this reaction, kinds of the solvent and the reagent and amounts thereof are preferably properly selected depending upon the substrates and reaction conditions employed for the reaction.

2) The compound of the present invention (1) wherein $R^5$ represents a hydrogen atom, which is referred to as compound (1b) of the present invention, can be produced from the compound (1a) of the present invention.

That is, the compound (1b) of the present invention can be produced by hydrolysis of the ester moiety of the compound (1a) of the present invention.

This reaction is an ordinary ester hydrolysis reaction to be conducted under an acidic or alkaline condition. The acidic condition is a condition wherein, for example, hydrochloric acid, sulfuric acid, acetic acid, trifluoroacetic acid, methanesulfonic acid, trifluoromethanesulfonic acid, phosphroc acid, polyphosphoric acid and the like are used alone or in any combination thereof. The alkaline condition is a condition wherein, for example, an alkali metal hydride such as lithium hydroxide, sodium hydroxide or potassium hydroxide, an alkali metal carbonate such as lithium carbonate, sodium carbonate or potassium carbonate, an alkali metal hydrogencarbonate such as sodium hydrogencarbonate or potassium hydrogencarbonate, or ammonia is used.

Also, this reaction can be conducted in the absence of or in a solvent. Examples of the solvent to be used include an alcohol such as methanol, ethanol, n-propanol, isopropanol, n-butanol, t-butanol, 2-methoxyethanol or ethylene glycol, an ether such as dioxane, tetrahydrofuran, dimethoxyethane, isopropyl ether or diethyl ether, a nitrile such as acetoni trile, a ketone such as acetone or methyl ethyl ketone, an aliphatic hydrocarbon such as petroleum ether, n-hexane or cyclohexane, an aromatic hydrocarbon such as benzene, toluene, xylene or chlorobenzene, an ester such as ethyl acetate or ethyl propionate, an alkyl halide such as dichloromethane, chloroform, tetrachlorocarbon or 1,2-dichloroethane, pyridine, N,N-dimethylformamide, dimethylsulfoxide, and water.

In this reaction, kinds of the solvent and the reagent and amounts thereof are preferably properly selected depending upon the substrates and reaction conditions employed for the reaction.

In using the carboxylic acid compound of the invention represented by the formula (1) or the pharmaceutically acceptable salt thereof for treating diseases wherein VEGF is involved, the compound of the invention can be administered orally or parenterally. The dosage forms of the same are tablets, capsules, granules, abstracts, powders, troches, ointments, creams, emulsions, suspensions, suppositories, injections, or the like, each of which may be produced according to the conventional formulation techniques (for example, methods defined in the 12th revised edition of the Japanese Pharmacopeia). These dosage forms may be appropriately selected depending on the conditions and ages of the patients, as well as the purpose of the treatment. Upon manufacturing preparations in various dosage forms, conventional excipients (for example, crystalline cellulose, starch, lactose, mannitol, or the like), binders (for example, hydroxypropylcellulose, polyvinylpyrrolidone, or the like), lubricants (for example, magnesium stearate, talc, or the like), disintegrants (for example, carboxymethylcellulose calcium, or the like) and the like may be employed. The dosage of the compound according to the present invention is in the range of from 1 to 2000 mg per day in a single dosage or divided into several dosages, in the case of an adult human subject to be treated. This dosage may vary appropriately depending on the age, weight, and condition of each individual patient.

## Best Mode for Carrying Out the Invention

Examples and Test Examples are given below to illustrate the invention in more detail.

Example 1:

Preparation of N-[3-{4-(1-octadecyloxy)phenyl}propionyl]-N-phenylglycine

A suspension of 3-{4-(1-octadecyloxy)phenyl}propionic acid (2.00 g, 4.78 mmol) and thionyl chloride (3.26 g, 274.00 mmol) in benzene (40 ml) was refluxed under heating for 1.5 hours. To a residue obtained by evaporating the solvent under reduced pressure was added dichloromethane (80 ml) and, after adding thereto successively aniline (0.60 g, 6.44 mmol) and triethylamine (1.45 g, 14.3 mmol) , the mixture was stirred at room temperature for 2 hours. The reaction solution was diluted with chloroform and washed with successive, 1M hydrochloric acid and saturated brine. The organic layer was dried over anhydrous magnesium sulfate, and the solvent was evaporated under reduced pressure. To the thus-obtained residue (1.0 g, 2.03 mmol) was added a mixed solution of tetrahydrofuran/N,N-dimethylformamide (5:1) (30ml) toprepare a solution, then 60% sodium hydride (0.12 g, 3.05 mmol) was added thereto at room temperature, followed by stirring the resultant mixture for 30 minutes. Subsequently, bromo tert-butyl acetate (0.48 g, 2.44 mmol) was dropwise added to the reaction solution, followed by stirring at room temperature for 14 hours. To the reaction solution was added a saturated ammonium chloride aqueous solution, and the mixture was extracted with ethyl acetate. The organic layer was washed with successive, water and saturated brine and, after drying over anhydrous magnesium sulfate, the solvent was evaporated under reduced pressure. Trifluoroacetic acid (20 ml) was added to the resultant residue, followed by stirring for one hour. After evaporating trifluoroacetic acid under reduced pressure, the thus-obtained residue was recrustallized from methanol to obtain N-[3-{4-(1-octadecyloxy)phenyl}propionyl-N-phenylglycine (0.91 g) as a colorless powder.
$^1$H-NMR (300MHz, $\delta$ ppm in $CDCl_3$)
0.88 (3H, t, J=7Hz), 1.25-1.79 (32H, m), 2.40 (2H, t, J=8Hz), 2.85 (2H, t, J=8Hz) , 3.89 (2H, t, J=7Hz), 4.39 (2H, s), 6.75-7.41 (9H, m)

Examples 2 to 113:

The compounds of the invention shown in Table 1 (compounds of Examples 2 to 113) were obtained from corresponding amino compounds and corresponding carboxylic acids in the same manner as in the compound of Example 1.

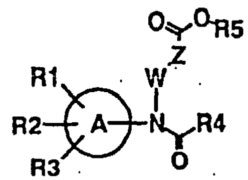

| Ex. No. | 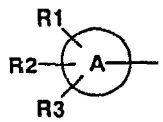 | 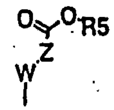 | —R4 | $^1$H NMR (300MHz, δ ppm in $CDCl_3$) |
|---|---|---|---|---|
| 1 | 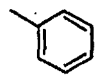 | $-CH_2COOH$ | 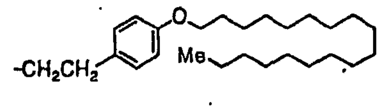 | 0.88 (3H, t, J=7Hz), 1.25-1.79 (32H, m), 2.40 (2H, m), 2.85 (2H, m), 3.89 (2H, t, J=7Hz), 4.39 (2H, s), 6.75-7.41 (9H, m). |
| 2 | 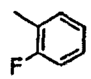 | $-CH_2COOH$ | 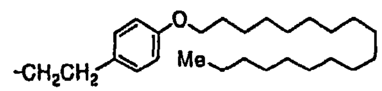 | 0.88 (3H, t, J=7Hz), 1.20-1.43 (30H, m), 1.73 (2H, m), 2.40 (2H, m), 2.86 (2H, m), 3.88 (2H, t, J=7Hz), 4.38 (2H, q, J=17Hz), 6.74-7.39 (8H, m). |
| 3 | 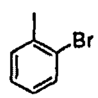 | $-CH_2COOH$ | 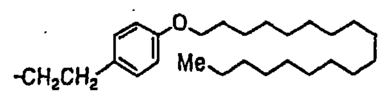 | 0.88 (3H, t, J=7Hz), 1.20-1.43 (30H, m), 1.74 (2H, m), 2.30 (2H, m), 2.86 (2H, m), 3.89 (2H, t, J=7Hz), 4.34 (2H, q, J=17Hz), 6.75-7.68 (8H, m). |
| 4 | 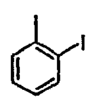 | $-CH_2COOH$ | 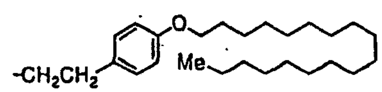 | 0.88 (3H, t, J=7Hz), 1.20-1.43 (30H, m), 1.74 (2H, m), 2.28 (2H, m), 2.87 (2H, m), 3.89 (2H, t, J=7Hz), 4.32 (2H, q, J=17Hz), 6.75-7.93 (8H, m). |
| 5 | 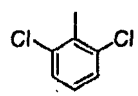 | $-CH_2COOH$ | 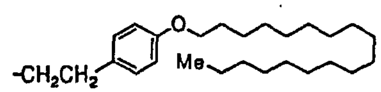 | 0.88 (3H, t, J=7Hz), 1.20-1.43 (30H, m), 1.74 (2H, m), 2.32 (2H, t, J=8Hz), 2.90 (2H, t, J=8Hz), 3.89 (2H, t, J=7Hz), 4.26 (2H, s), 6.75-7.46 (7H, m). |

| Ex. No. | 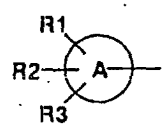 | 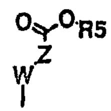 | —R4 | $^1$H NMR (300MHz, δ ppm in $CDCl_3$) |
|---|---|---|---|---|
| 6 | 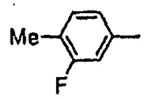 | $-CH_2COOH$ | 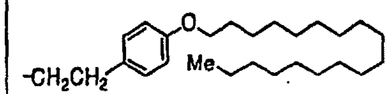 | 0.88 (3H, t, J=7Hz), 1.20-1.53 (30H, m), 1.75 (2H, m), 2.27 (3H, s), 2.41 (2H, t, J=8Hz), 2.86 (2H, t, J=8Hz), 3.90 (2H, t, J=7Hz), 4.35 (2H, s), 6.76-7.19 (7H, m). |
| 7 | 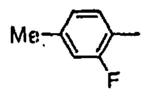 | $-CH_2COOH$ | 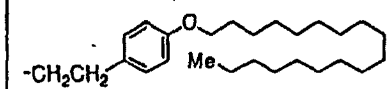 | 0.88 (3H, t, J=7Hz), 1.20-1.43 (30H, m), 1.74 (2H, m), 2.36 (3H, s), 2.39 (2H, m), 2.85 (2H, m), 3.89 (2H, t, J=7Hz), 4.36 (2H, q, J=17Hz), 6.74-7.22 (7H, m). |
| 8 | 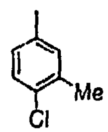 | $-CH_2COOH$ | 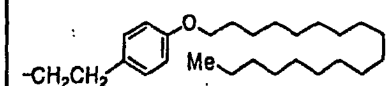 | 0.88 (3H, t, J=7Hz), 1.20-1.48 (30H, m), 1.75 (2H, m), 2.33 (3H, s), 2.39 (2H, t, J=8Hz), 2.86 (2H, t, J=8Hz), 3.90 (2H, t, J=7Hz), 4.34 (2H, s), 6.76-7.33 (7H, m). |
| 9 | 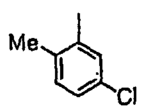 | $-CH_2COOH$ | 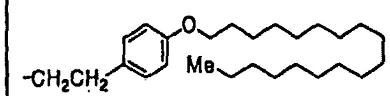 | 0.88 (3H, t, J=7Hz), 1.20-1.43 (30H, m), 1.75 (2H, m), 2.09 (3H, s), 2.16-2.42 (2H, m), 2.87 (2H, t, J=8Hz), 3.90 (2H, t, J=7Hz), 4.25 (2H, q, J=17Hz), 6.76-7.26 (7H, m). |
| 10 | 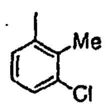 | $-CH_2COOH$ | 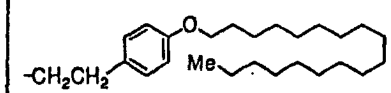 | 0.88 (3H, t, J=7Hz), 1.20-1.48 (30H, m), 1.75 (2H, m), 2.17 (3H, s), 2.18-2.42 (2H, m), 2.86 (2H, t, J=8Hz), 3.90 (2H, t, J=7Hz), 4.26 (2H, q, J=17Hz), 6.75-7.40 (7H, m). |
| 11 | 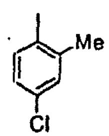 | $-CH_2COOH$ | 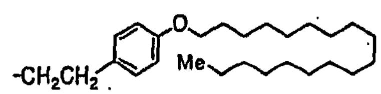 | 0.88 (3H, t, J=7Hz), 1.20-1.43 (30H, m), 1.75 (2H, m), 2.09 (3H, s), 2.16-2.40 (2H, m), 2.85 (2H, t, J=7Hz), 3.90 (2H, t, J=7Hz), 4.24 (2H, q, J=17Hz), 6.75-7.25 (7H, m). |

| Ex. No. | 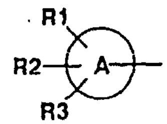 | 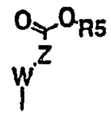 | —R4 | $^1$H NMR (300MHz, δ ppm in $CDCl_3$) |
|---|---|---|---|---|
| 12 | 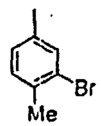 | $-CH_2COOH$ | 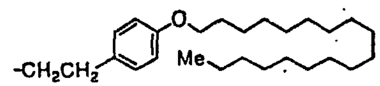 | 0.88 (3H, t, J=7Hz), 1.20-1.43 (30H, m), 1.75 (2H, m), 2.39 (3H, s), 2.40 (2H, t, J=8Hz), 2.86 (2H, t, J=8Hz), 3.91 (2H, t, J=7Hz), 4.34 (2H, s), 6.77-7.32 (7H, m). |
| 13 | 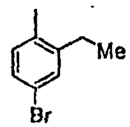 | $-CH_2COOH$ | 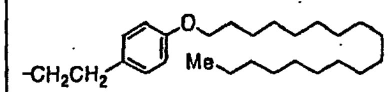 | 0.88 (3H, t, J=7Hz), 1.13-1.52 (33H, m), 1.75 (2H, m), 2.13-2.50 (4H, m), 2.85 (2H, t, J=7Hz), 3.90 (2H, t, J=7Hz), 4.24 (2H, q, J=17Hz), 6.75-7.44 (7H, m). |
| 14 | 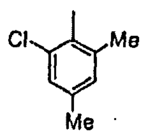 | $-CH_2COOH$ | 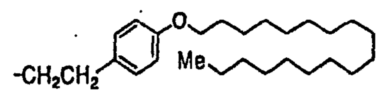 | 0.88 (3H, t, J=7Hz), 1.20-1.43 (30H, m), 1.74 (2H, m), 2.13 (3H, s), 2.18-2.37 (2H, m), 2.31 (3H, s), 2.84-2.92 (2H, m), 3.89 (2H, t, J=7Hz), 4.20 (2H, q, J=16Hz), 6.74-7.14 (6H, m). |
| 15 | 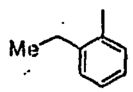 | $-CH_2COOH$ | 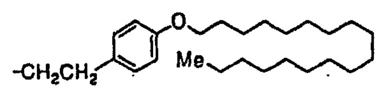 | 0.88 (3H, t, J=7Hz), 1.14-1.43 (33H, m), 1.74 (2H, m), 2.16-2.54 (4H, m), 2.86 (2H, t, J=7Hz), 3.89 (2H, t, J=7Hz), 4.29 (2H, q, J=17Hz), 6.72-7.33 (8H, m). |
| 16 | 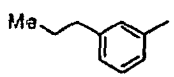 | $-CH_2COOH$ | 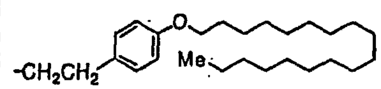 | 0.86-0.95 (6H, m), 1.25-1.79 (34H, m), 2.41 (2H, t, J=8Hz), 2.56 (2H, t, J=7Hz), 2.85 (2H, t, J=7Hz), 3.89 (2H, t, J=7Hz), 4.37 (2H, s), 6.74-7.29 (8H, m). |
| 17 | 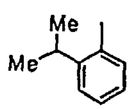 | $-CH_2COOH$ | 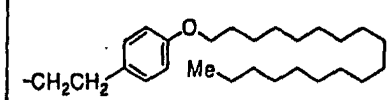 | 0.88 (3H, t, J=7Hz), 1.14-1.43 (36H, m), 1.74 (2H, m), 2.16-2.43 (2H, m), 2.80-3.01 (3H, m), 3.88 (2H, t, J=7Hz), 4.29 (2H, q, J=17Hz), 6.73-7.36 (8H, m). |

| Ex. No. | 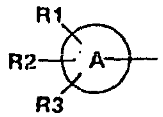 | 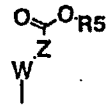 | —R4 | $^1$H NMR (300MHz, δ ppm in $CDCl_3$) |
|---|---|---|---|---|
| 18 | 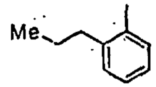 | $-CH_2COOH$ | 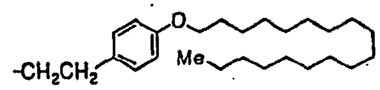 | 0.86-0.95 (6H, m), 1.20-1.78 (34H, m), 2.15-2.45 (4H, m), 2.85 (2H, t, J=7Hz), 3.88 (2H, t, J=7Hz), 4.28 (2H, q, J=17Hz), 6.72-7.31 (8H, m). |
| 19 | 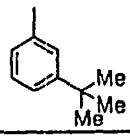 | $-CH_2COOH$ | 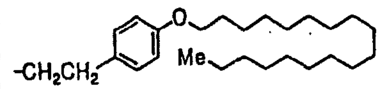 | 0.88 (3H, t, J=7Hz), 1.20-1.43 (39H, m), 1.75 (2H, m), 2.40 (2H, t, J=8Hz), 2.85 (2H, t, J=8Hz), 3.89 (2H, t, J=7Hz), 4.39 (2H, s), 6.74-7.29 (8H, m). |
| 20 | 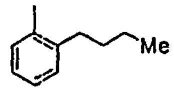 | $-CH_2COOH$ | 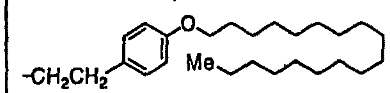 | 0.86-0.96 (6H, m), 1.17-1.56 (30H, m), 1.74 (2H, m), 2.11-2.43 (6H, m), 2.85 (2H, t, J=8Hz), 3.89 (2H, t, J=7Hz), 4.28 (2H, q, J=17Hz), 6.74-7.31 (8H, m). |
| 21 | 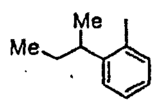 | $-CH_2COOH$ | 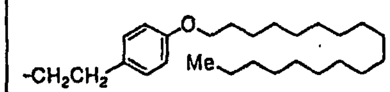 | 0.78-0.90 (6H, m), 1.10-1.85 (34H, m), 2.12-2.40 (2H, m), 2.68-2.93 (3H, m), 3.88 (2H, t, J=7Hz), 4.28 (2H, q, J=17Hz), 6.73-7.35 (8H, m). |
| 22 | 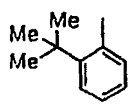 | $-CH_2COOH$ | 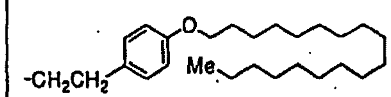 | 0.88 (3H, t, J=7Hz), 1.20-1.43 (39H, m), 1.75 (2H, m), 2.23-2.43 (2H, m), 2.78-2.98 (2H, m), 3.88 (2H, t, J=7Hz), 4.28 (2H, q, J=17Hz), 6.73-7.32 (8H, m). |
| 23 | 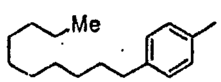 | $-CH_2COOH$ | 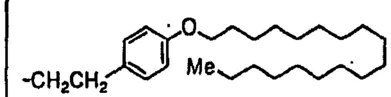 | 0.88 (6H, t, J=7Hz), 1.20-1.77 (48H, m), 2.40 (2H, t, J=8Hz), 2.60 (2H, t, J=7Hz), 2.85 (2H, t, J=7Hz), 3.89 (2H, t, J=7Hz), 4.37 (2H, s), 6.74-7.18 (8H, m). |

| Ex. No. | 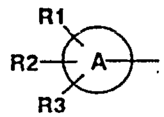 | 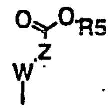 | —R4 | $^1$H NMR (300MHz, δ ppm in $CDCl_3$) |
|---|---|---|---|---|
| 24 | 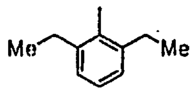 | $-CH_2COOH$ | 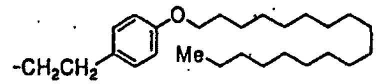 | 0.88 (3H, t, J=7Hz), 1.14-1.43 (36H, m), 1.74 (2H, m), 2.22 (2H, t, J=8Hz), 2.31-2.51 (4H, m), 2.85 (2H, t, J=8Hz), 3.89 (2H, t, J=7Hz), 4.21 (2H, s), 6.74-7.33 (7H, m). |
| 25 | 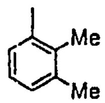 | $-CH_2COOH$ | 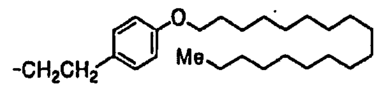 | 0.88 (3H, t, J=7Hz), 1.20-1.43 (30H, m), 1.74 (2H, m), 2.03 (3H, s), 2.16-2.43 (2H, m), 2.28 (3H, s), 2.85 (2H, t, J=8Hz), 3.89 (2H, t, J=7Hz), 4.27 (2H, q, J=16Hz), 6.75-7.16 (7H, m). |
| 26 | 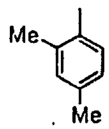 | $-CH_2COOH$ | 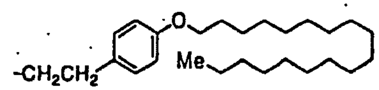 | 0.88 (3H, t, J=7Hz), 1.20-1.43 (30H, m), 1.74 (2H, m), 2.08 (3H, s), 2.18-2.43 (2H, m), 2.32 (3H, s), 2.85 (2H, t, J=8Hz), 3.89 (2H, t, J=8Hz), 4.26 (2H, q, J=16Hz), 6.74-7.09 (7H, m). |
| 27 | 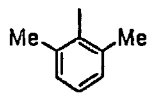 | $-CH_2COOH$ | 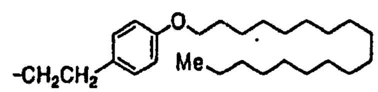 | 0.88 (3H, t, J=7Hz), 1.20-1.43 (30H, m), 1.74 (2H, m), 2.10 (3H, s), 2.26 (2H, t, J=8Hz), 2.87 (2H, t, J=8Hz), 3.89 (2H, t, J=7Hz), 4.19 (2H, s), 6.74-7.20 (7H, m). |
| 28 | 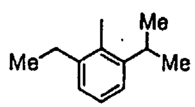 | $-CH_2COOH$ | 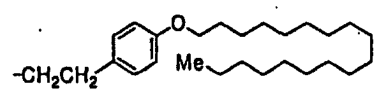 | 0.88 (3H, t, J=7Hz), 1.13-1.43 (39H, m), 1.74 (2H, m), 2.20-2.50 (4H, m), 2.82-2.95 (3H, m), 3.88 (2H, t, J=7Hz), 4.21 (2H, s), 6.74-7.36 (7H, m). |
| 29 | 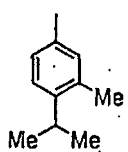 | $-CH_2COOH$ | 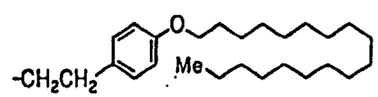 | 0.88 (3H, t, J=7Hz), 1.20-1.43 (36H, m), 1.75 (2H, m), 2.29 (3H, s), 2.42 (2H, t, J=8Hz), 2.86 (2H, t, J=8Hz), 3.11 (1H, m), 3.90 (2H, t, J=7Hz), 4.35 (2H, s), 6.75-7.22 (7H, m). |

| Ex. No. | 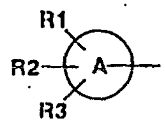 | 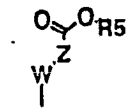 | —R4 | $^1$H NMR (300MHz, δ ppm in $CDCl_3$) |
|---|---|---|---|---|
| 30 | 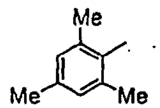 | $-CH_2COOH$ | 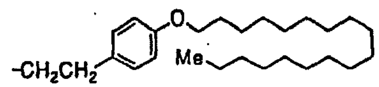 | 0.88 (3H, t, J=7Hz), 1.08-1.50 (30H, m), 1.74 (2H, m), 2.05 (6H, s), 2.26 (2H, t, J=7Hz), 2.28 (3H, s), 2.87 (2H, t, J=7Hz), 3.89 (2H, t, J=7Hz), 4.28 (2H, s), 6.76 (2H, m), 6.90 (2H, s), 6.98 (2H, m). |
| 31 | 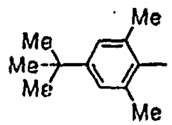 | $-CH_2COOH$ | 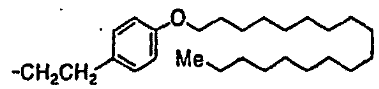 | 0.88 (3H, t, J=7Hz), 1.20-1.43 (39H, m), 1.74 (2H, m), 2.08 (6H, s), 2.27 (2H, t, J=8Hz), 2.87 (2H, t, J=8Hz), 3.89 (2H, t, J=7Hz), 4.18 (2H, s), 6.75-7.07 (6H, m). |
| 32 | 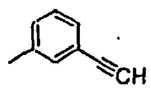 | $-CH_2COOH$ | 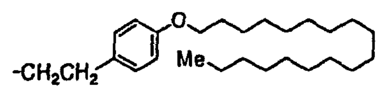 | 0.88 (3H, t, J=7Hz), 1.20-1.43 (30H, m), 1.74 (2H, m), 2.39 (2H, t, J=8Hz), 2.85 (2H, t, J=8Hz), 3.13 (1H, s), 3.90 (2H, t, J=7Hz), 4.36 (2H, s), 6.74-7.47 (8H, m). |
| 33 | 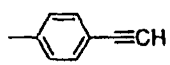 | $-CH_2COOH$ | 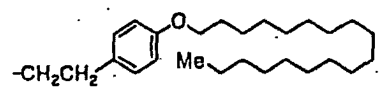 | 0.88 (3H, t, J=7Hz), 1.20-1.43 (30H, m), 1.75 (2H, m), 2.41 (2H, m), 2.89 (2H, t, J=8Hz), 3.90 (2H, t, J=7Hz), 4.36 (2H, s), 6.72-7.49 (8H, m). |
| 34 | 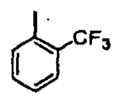 | $-CH_2COOH$ | 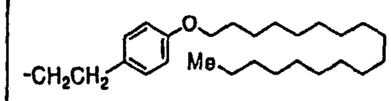 | 0.88 (3H, t, J=7Hz), 1.20-1.43 (30H, m), 1.74 (2H, m), 2.25 (2H, t, J=8Hz), 2.76-2.95 (2H, m), 3.89 (2H, t, J=7Hz), 4.32 (2H, q, J=17Hz), 6.74-7.76 (8H, m). |
| 35 | 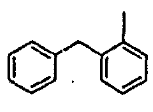 | $-CH_2COOH$ | 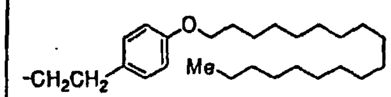 | 0.88 (3H, t, J=7Hz), 1.20-1.44 (30H, m), 1.73 (2H, m), 1.89 (1H, m), 2.23 (1H, m), 2.60-2.83 (2H, m), 3.77 (2H, s), 3.88 (2H, t, J=7Hz), 4.03 (2H, q, J=17Hz), 6.73-7.36 (13H, m). |

| Ex. No. | 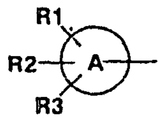 | 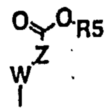 | —R4 | $^1$H NMR (300MHz, δ ppm in $CDCl_3$) |
|---|---|---|---|---|
| 36 | 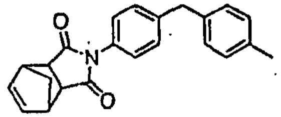 | $-CH_2COOH$ | 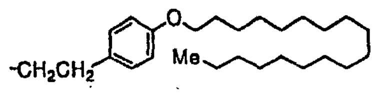 | 0.88 (3H, t, J=7Hz), 1.20-1.80 (34H, m), 2.41 (2H, t, J=8Hz), 2.84 (2H, t, J=8Hz), 3.42 (2H, d, J=2Hz), 3.43 (2H, d, J=1Hz), 3.89 (2H, t, J=7Hz), 3.97 (2H, s), 4.36 (2H, s), 6.25 (2H, s), 6.74-7.26 (12H, m). |
| 37 | 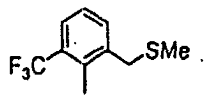 | $-CH_2COOH$ | 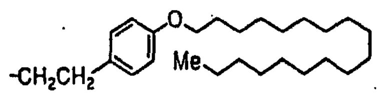 | 0.88 (3H, t, J=7Hz), 1.20-1.43 (30H, m), 1.73 (2H, m), 2.05 (3H, s), 2.20 (2H, m), 2.85 (2H, m), 3.75 (2H, q, J=12Hz), 3.88 (2H, t, J=7Hz), 4.24 (2H, q, J=17Hz), 6.73-7.68 (7H, m). |
| 38 | 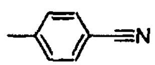 | $-CH_2COOH$ | 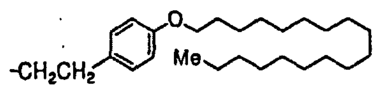 | 0.88 (3H, t, J=7Hz), 1.20-1.47 (30H, m), 1.75 (2H, m), 2.39 (2H, t, J=8Hz), 2.86 (2H, t, J=8Hz), 3.90 (2H, t, J=7Hz), 4.37 (2H, s), 6.75-7.69 (8H, m). |
| 39 | 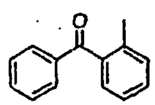 | $-CH_2COOH$ | 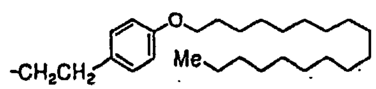 | 0.88 (3H, t, J=7Hz), 1.20-1.43 (30H, m), 1.75 (2H, m), 2.34 (2H, m), 2.62-2.93 (2H, m), 3.89 (2H, m), 4.26 (2H, q, J=17Hz), 6.71-7.76 (13H, m). |
| 40 | 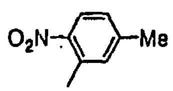 | $-CH_2COOH$ | 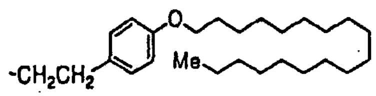 | 0.88 (3H, t, J=7Hz), 1.20-1.43 (30H, m), 1.74 (2H, m), 2.28 (2H, m), 2.40 (3H, s), 3.89 (2H, t, J=7Hz), 4.34 (2H, q, J=17Hz), 6.72-7.93 (7H, m). |
| 41 | 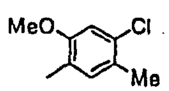 | $-CH_2COOH$ | 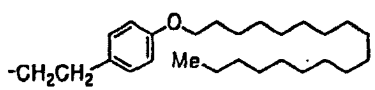 | 0.88 (3H, t, J=7Hz), 1.20-1.43 (30H, m), 1.74 (2H, m), 2.31 (2H, m), 2.25 (3H, s), 2.31 (2H, m), 2.84 (2H, m), 3.77 (3H, s), 3.89 (2H, t, J=7Hz), 4.28 (2H, q, J=17Hz), 6.75-7.03 (6H, m). |

| Ex. No. | 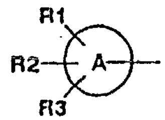 | 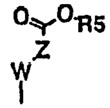 | —R4 | $^1H$ NMR (300MHz, δ ppm in $CDCl_3$) |
|---|---|---|---|---|
| 42 | 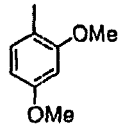 | $-CH_2COOH$ | 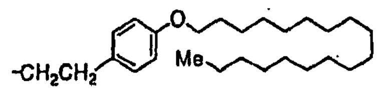 | 0.88 (3H, t, J=7Hz), 1.20-1.43 (30H, m), 1.74 (2H, m), 2.34 (2H, m), 2.83 (2H, m), 3.77 (3H, s), 3.81 (3H, s), 3.88 (2H, t, J=7Hz), 4.26 (2H, q, J=17Hz), 6.41-7.15 (7H, m). |
| 43 | 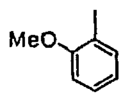 | $-CH_2COOH$ | 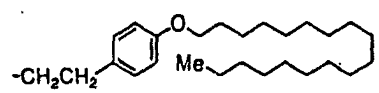 | 0.88 (3H, t, J=7Hz), 1.20-1.43 (30H, m), 1.74 (2H, m), 2.25-2.48 (2H, m), 2.84 (2H, m), 3.81 (3H, s), 3.89 (2H, t, J=7Hz), 4.31 (2H, q, J=17Hz), 6.74-7.36 (8H, m). |
| 44 | 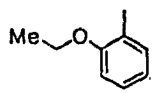 | $-CH_2COOH$ | 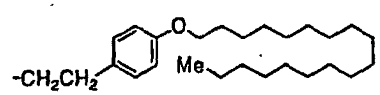 | 0.88 (3H, t, J=7Hz), 1.20-1.43 (33H, m), 1.74 (2H, m), 2.26-2.46 (2H, m), 2.84 (2H, m), 3.89 (2H, t, J=7Hz), 4.05 (2H, q, J=7Hz), 4.31 (2H, q, J=17Hz), 6.73-7.34 (8H, m). |
| 45 | 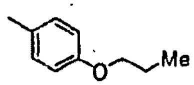 | $-CH_2COOH$ | 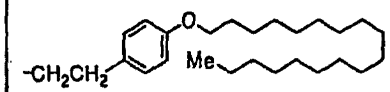 | 0.88 (3H, t, J=7Hz), 1.04 (3H, t, J=7Hz), 1.20-1.48 (30H, m), 1.70-1.87 (4H, m), 2.39 (2H, t, J=8Hz), 2.85 (2H, t, J=8Hz), 3.87-3.93 (4H, m), 4.35 (2H, s), 6.75-7.10 (8H, m). |
| 46 | 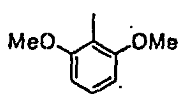 | $-CH_2COOH$ | 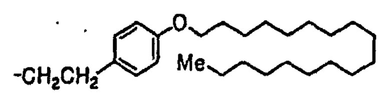 | 0.88 (3H, t, J=7Hz), 1.20-1.43 (39H, m), 1.75 (2H, m), 2.31 (2H, t, J=8Hz), 2.83 (2H, t, J=8Hz), 3.81 (6H, s), 3.89 (2H, t, J=7Hz), 3.81 (6H, s), 4.20 (2H, s), 6.59-7.31 (7H, m). |
| 47 | 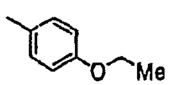 | $-CH_2COOH$ | 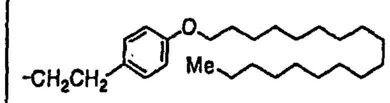 | 0.88 (3H, t, J=7Hz), 1.20-1.48 (33H, m), 1.75 (2H, m), 2.39 (2H, t, J=8Hz), 2.84 (2H, t, J=7Hz), 3.90 (2H, t, J=7Hz), 4.02 (2H, q, J=7Hz), 4.35 (2H, s), 6.74-7.10 (8H, m). |

| Ex. No. | 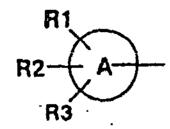 | 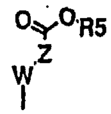 | —R4 | $^1$H NMR (300MHz, δ ppm in $CDCl_3$) |
|---|---|---|---|---|
| 48 | 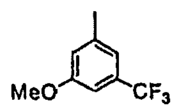 | $-CH_2COOH$ | 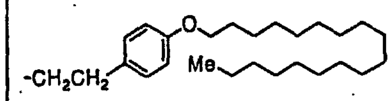 | 0.88 (3H, t, J=7Hz), 1.20-1.43 (30H, m), 1.75 (2H, m), 2.41 (2H, t, J=8Hz), 2.87 (2H, t, J=8Hz), 3.82 (3H, s), 3.90 (2H, t, J=7Hz), 4.36 (2H, s), 6.76-7.10 (7H, m). |
| 49 | 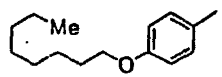 | $-CH_2COOH$ | 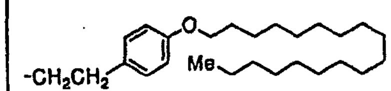 | 0.86-0.90 (6H, m), 1.20-1.43 (40H, m), 1.75 (4H, m), 2.39 (2H, m), 2.85 (2H, m), 3.91 (4H, m), 4.35 (2H, s), 6.75-7.08 (8H, m). |
| 50 | 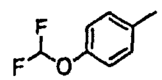 | $-CH_2COOH$ | 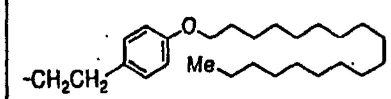 | 0.88 (3H, t, J=7Hz), 1.20-1.43 (30H, m), 1.75 (2H, m), 2.38 (2H, t, J=8Hz), 2.86 (2H, t, J=8Hz), 3.90 (2H, t, J=7Hz), 4.36 (2H, s), 6.40 (1H, d, J=73Hz), 6.75-7.20 (8H, m). |
| 51 | 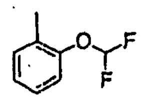 | $-CH_2COOH$ | 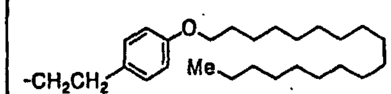 | 0.88 (3H, t, J=7Hz), 1.20-1.43 (30H, m), 1.74 (2H, m), 2.36 (2H, t, J=8Hz), 2.86 (2H, t, J=8Hz), 3.89 (2H, t, J=7Hz), 4.34 (2H, q, J=17Hz), 6.36 (1H, d, J=73Hz), 6.72-7.40 (8H, m). |
| 52 | 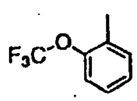 | $-CH_2COOH$ | 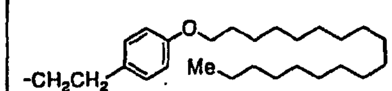 | 0.88 (3H, t, J=7Hz), 1.20-1.43 (30H, m), 1.74 (2H, m), 2.11-2.38 (2H, m), 2.81-2.93 (2H, m), 3.89 (2H, t, J=7Hz), 4.34 (2H, q, J=17Hz), 6.75-7.42 (8H, m). |
| 53 | 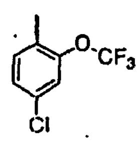 | $-CH_2COOH$ | 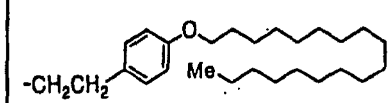 | 0.88 (3H, t, J=7Hz), 1.20-1.43 (30H, m), 1.74 (2H, m), 2.23 (2H, m), 2.89 (2H, m), 3.90 (2H, t, J=7Hz), 4.31 (2H, q, J=17Hz), 6.76-7.36 (7H, m). |

| Ex. No. | 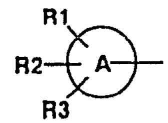 | 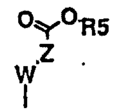 | —R4 | $^1$H NMR (300MHz, δ ppm in $CDCl_3$) |
|---|---|---|---|---|
| 54 | 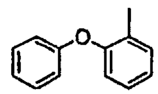 | $-CH_2COOH$ | 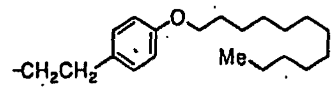 | 0.88 (3H, t, J=7Hz), 1.00-1.52 (18H, m), 1.75 (2H, m), 2.47 (2H, m), 2.84 (2H, m), 3.90 (2H, t, J=7Hz), 3.92 (1H, d, J=17Hz), 4.72 (1H, d, J=17Hz), 6.76 (2H, m), 6.89-7.37 (11H, m). |
| 55 | 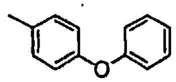 | $-CH_2COOH$ | 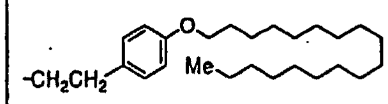 | 0.88 (3H, t, J=7Hz), 1.20-1.43 (30H, m), 1.74 (2H, m), 2.42 (2H, t, J=8Hz), 2.86 (2H, t, J=8Hz), 3.89 (2H, t, J=7Hz), 4.37 (2H, s), 6.75-7.41 (13H, m). |
| 56 | 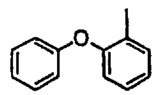 | $-CH_2COOH$ | 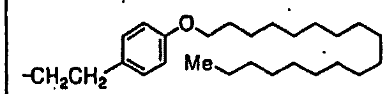 | 0.88 (3H, t, J=7Hz), 1.20-1.43 (30H, m), 1.75 (2H, m), 2.45 (2H, m), 2.84 (2H, m), 3.89 (2H, t, J=7Hz), 4.32 (2H, q, J=17Hz), 6.75-7.37 (13H, m). |
| 57 | 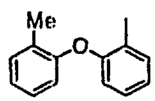 | $-CH_2COOH$ | 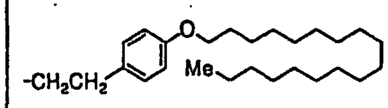 | 0.88 (3H, t, J=7Hz), 1.20-1.45 (30H, m), 1.74 (2H, m), 2.18 (3H, s), 2.50 (2H, m), 2.88 (2H, m), 3.89 (3H, t, J=7Hz), 4.40 (2H, q, J=17Hz), 6.69-7.34 (12H, m). |
| 58 | 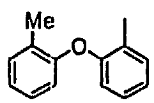 | $-CH_2COOH$ | 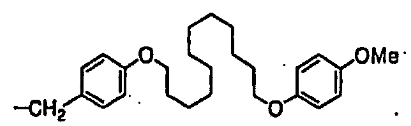 | 1.29-1.46 (16H, m), 1.74 (4H, m), 2.19 (3H, s), 3.56 (2H, d, J=2Hz), 3.76 (3H, s), 3.88-3.93 (4H, m), 4.41 (2H, q, J=17Hz), 6.69-7.43 (16H, m). |
| 59 | 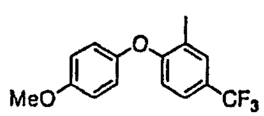 | $-CH_2COOH$ | 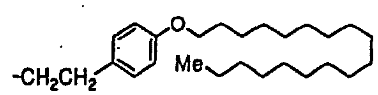 | 0.88 (3H, t, J=7Hz), 1.20-1.43 (30H, m), 1.75 (2H, m), 2.45 (2H, m), 2.90 (2H, t, J=8Hz), 3.82 (3H, s), 3.89 (2H, t, J=7Hz), 4.41 (2H, q, J=18Hz), 6.76-7.63 (11H, m). |

| Ex. No. | 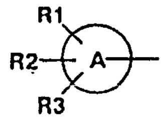 | 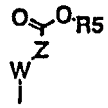 | —R4 | $^1$H NMR (300MHz, δ ppm in $CDCl_3$) |
|---|---|---|---|---|
| 60 | 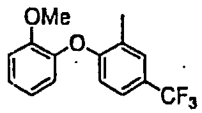 | $-CH_2COOH$ | 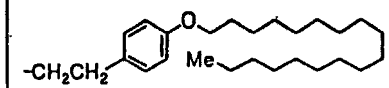 | 0.89 (3H, t, J=7Hz), 1.20-1.44 (30H, m), 1.75 (2H, m), 2.53 (2H, t, J=7Hz), 2.92 (2H, t, J=8Hz), 3.64 (3H, s), 3.90 (2H, t, J=7Hz), 4.51 (2H, q, J=17Hz), 6.66-7.64 (11H, m). |
| 61 | 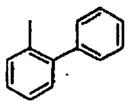 | $-CH_2COOH$ | 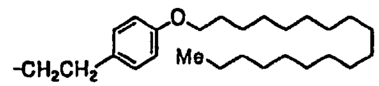 | 0.88 (3H, t, J=7Hz), 1.20-1.48 (30H, m), 1.76 (2H, m), 2.29-2.88 (4H, m), 3.91 (2H, t, J=7Hz), 3.94 (2H, q, J=17Hz), 6.76-7.45 (13H, m). |
| 62 | 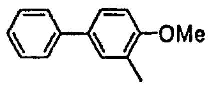 | $-CH_2COOH$ | 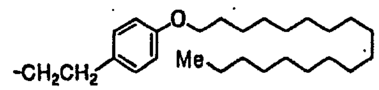 | 0.88 (3H, t, J=7Hz), 1.20-1.43 (30H, m), 1.73 (2H, m), 2.41 (2H, m), 2.86 (2H, m), 3.84 (3H, s), 3.85 (2H, t, J=7Hz), 4.36 (2H, q, J=17Hz), 6.70-7.58 (12H, m). |
| 63 | 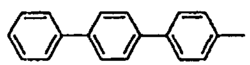 | $-CH_2COOH$ | 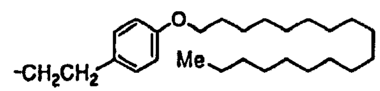 | 0.88 (3H, t, J=7Hz), 1.20-1.43 (30H, m), 1.74 (2H, m), 2.49 (2H, t, J=8Hz), 2.90 (2H, t, J=8Hz), 3.90 (2H, t, J=7Hz), 4.43 (2H, s), 6.76-7.71 (17H, m). |
| 64 | 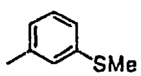 | $-CH_2COOH$ | 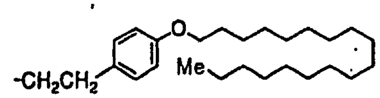 | 0.88 (3H, t, J=7Hz), 1.20-1.43 (30H, m), 1.74 (2H, m), 2.41 (2H, t, J=7Hz), 2.45 (3H, s), 2.86 (2H, t, J=7Hz), 3.90 (2H, t, J=7Hz), 4.36 (2H, s), 6.75-7.30 (8H, m). |
| 65 | 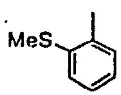 | $-CH_2COOH$ | 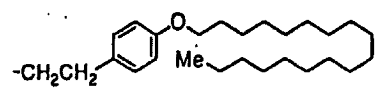 | 0.88 (3H, t, J=7Hz), 1.20-1.43 (30H, m), 1.74 (2H, m), 2.29-2.37 (2H, m), 2.43 (3H, s), 2.87 (2H, m), 3.88 (2H, t, J=7Hz), 4.32 (2H, q, J=17Hz), 6.95-7.88 (8H, m). |

| Ex. No. | 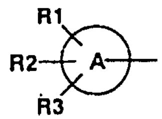 | 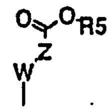 | —R4 | $^1H$ NMR (300MHz, δ ppm in $CDCl_3$) |
|---|---|---|---|---|
| 66 | 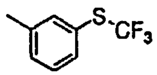 | $-CH_2COOH$ | 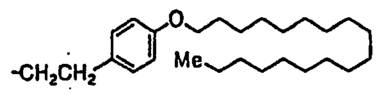 | 0.88 (3H, t, J=7Hz), 1.20-1.45 (30H, m), 1.75 (2H, m), 2.38 (2H, t, J=8Hz), 2.85 (2H, t, J=8Hz), 3.90 (2H, t, J=7Hz), 4.38 (2H, s), 6.75-7.65 (8H, m). |
| 67 | 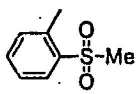 | $-CH_2COOH$ | 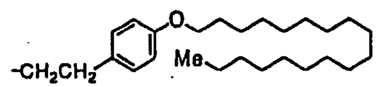 | 0.88 (3H, t, J=7Hz), 1.20-1.43 (30H, m), 1.74 (2H, m), 2.15-2.48 (2H, m), 2.84 (3H, s), 2.90 (2H, m), 3.88 (2H, t, J=7Hz), 4.46 (2H, q, J=18Hz), 6.73-8.14 (8H, m). |
| 68 | 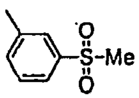 | $-CH_2COOH$ | 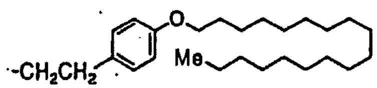 | 0.88 (3H, t, J=7Hz), 1.20-1.43 (30H, m), 1.75 (2H, m), 2.38 (2H, t, J=8Hz), 2.87 (2H, t, J=8Hz), 3.07 (3H, s), 3.90 (2H, t, J=7Hz), 4.41 (2H, s), 6.76-7.94 (8H, m). |
| 69 | 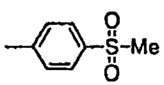 | $-CH_2COOH$ | 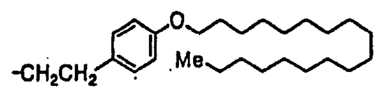 | 0.88 (3H, t, J=7Hz), 1.20-1.43 (30H, m), 1.75 (2H, m), 2.41 (2H, t, J=7Hz), 2.87 (2H, t, J=8Hz), 3.08 (3H, s), 3.90 (2H, t, J=7Hz), 4.39 (2H, s), 6.76-7.93 (8H, m). |
| 70 | 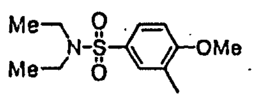 | $-CH_2COOH$ | 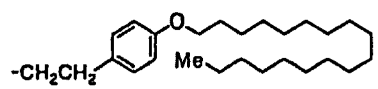 | 0.88 (3H, t, J=7Hz), 1.20-1.43 (30H, m), 1.74 (2H, m), 2.19-2.43 (2H, m), 2.83 (2H, t, J=8Hz), 3.18 (4H, q, J=7Hz), 3.87 (3H, s), 3.88 (2H, t, J=7Hz), 4.34 (2H, q, J=17Hz), 6.73-7.82 (7H, m). |
| 71 | 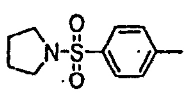 | $-CH_2COOH$ | 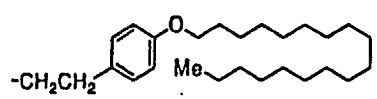 | 0.88 (3H, t, J=7Hz), 1.20-1.43 (30H, m), 1.70-1.85 (6H, m), 2.40 (2H, t, J=8Hz), 2.86 (2H, t, J=8Hz), 3.27 (4H, t, J=7Hz), 3.90 (2H, t, J=7Hz), 4.39 (2H, s), 6.75-7.86 (8H, m). |

| Ex. No. | 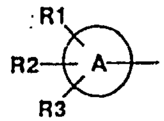 | 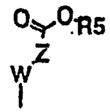 | —R4 | $^1$H NMR (300MHz, δ ppm in $CDCl_3$) |
|---|---|---|---|---|
| 72 | 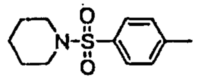 | $-CH_2COOH$ | 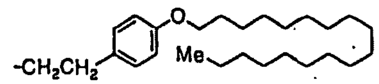 | 0.88 (3H, t, J=7Hz), 1.20-1.80 (38H, m), 2.40 (2H, t, J=8Hz), 2.86 (2H, t, J=7Hz), 3.03 (4H, t, J=5Hz), 3.90 (2H, t, J=7Hz), 4.39 (2H, s), 6.74-7.78 (8H, m). |
| 73 | 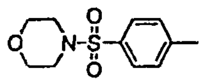 | $-CH_2COOH$ | 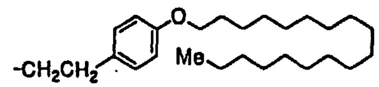 | 0.88 (3H, t, J=7Hz), 1.20-1.43 (30H, m), 1.75 (2H, m), 3.91 (2H, t, J=7Hz), 4.40 (2H, s), 6.77-7.77 (8H, m). |
| 74 | 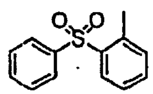 | $-CH_2COOH$ | 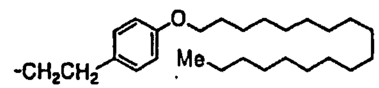 | 0.88 (3H, t, J=7Hz), 1.02-1.43 (31H, m), 1.68-1.86 (3H, m), 2.26 (1H, m), 2.68 (1H, m), 3.87 (2H, t, J=7Hz), 4.24 (2H, q, J=18Hz), 6.69-8.37 (13H, m). |
| 75 | 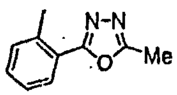 | $-CH_2COOH$ | 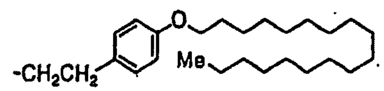 | 0.88 (3H, t, J=7Hz), 1.20-1.43 (30H, m), 1.74 (2H, m), 2.23 (2H, m), 2.59 (3H, s), 2.80 (2H, m), 3.88 (2H, t, J=7Hz), 4.32 (2H, q, J=17Hz), 6.69-8.08 (8H, m). |
| 76 | 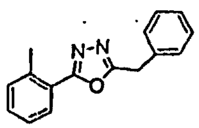 | $-CH_2COOH$ | 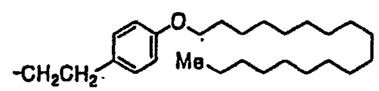 | 0.88 (3H, t, J=7Hz), 1.20-1.43 (30H, m), 1.73 (2H, m), 2.18 (2H, m), 2.85 (2H, m), 3.87 (2H, t, J=7Hz), 4.25 (2H, q, J=17Hz9, 6.67-8.02 (13H, m). |
| 77 | 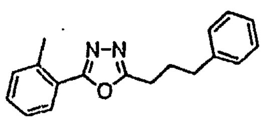 | $-CH_2COOH$ | 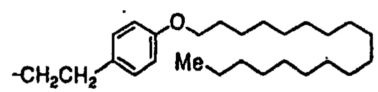 | 0.88 (3H, t, J=7Hz), 1.20-1.43 (30H, m), 1.73 (2H, m), 2.11-2.34 (4H, m), 2.69-2.93 (6H, m), 3.86 (2H, t, J=7Hz), 4.31 (2H, q, J=17Hz), 6.68-8.05 (13H, m). |

| Ex. No. | 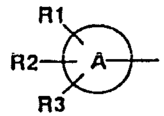 | 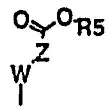 | —R4 | $^1$H NMR (300MHz, δ ppm in $CDCl_3$) |
|---|---|---|---|---|
| 78 | 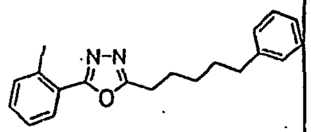 | $-CH_2COOH$ | 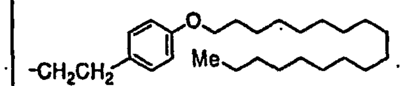 | 0.88 (3H, t, J=7Hz), 1.20-1.91 (38H, m), 2.23 (2H, m), 2.61-2.91 (6H, m), 3.87 (2H, t, J=7Hz), 4.31 (2H, q, J=17Hz), 6.67-8.07 (13H, m). |
| 79 | 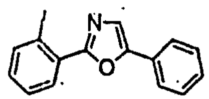 | $-CH_2COOH$ | 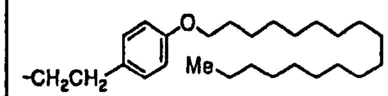 | 0.88 (3H, t, J=7Hz), 1.20-1.43 (30H, m), 1.71 (2H, m), 2.14-2.29 (2H, m), 2.75 (2H, m), 3.82 (2H, t, J=7Hz), 4.34 (2H, q, J=16Hz), 6.63-8.22 (14H, m). |
| 80 | 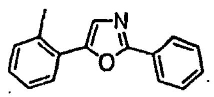 | $-CH_2COOH$ | 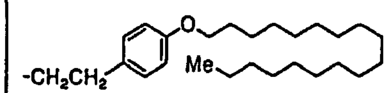 | 0.88 (3H, t, J=7Hz), 1.20-1.43 (30H, m), 1.70 (2H, m), 2.24-2.52 (2H, m), 2.85 (2H, m), 3.82 (2H, t, J=7Hz), 4.36 (2H, q, J=17Hz), 6.66-8.09 (14H, m). |
| 81 | 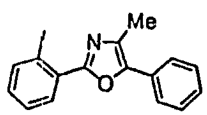 | $-CH_2COOH$ | 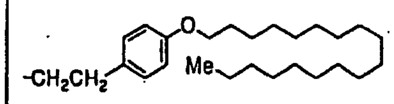 | 0.88 (3H, t, J=7Hz), 1.20-1.43 (30H, m), 1.71 (2H, m), 2.19 (2H, m), 2.44 (3H, s), 2.74 (2H, m), 3.79 (2H, m), 4.31 (2H, q, J=16Hz), 6.62-8.18 (13H, m). |
| 82 | 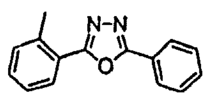 | $-CH_2COOH$ | 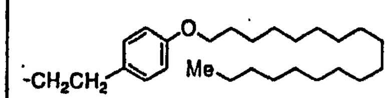 | 0.88 (3H, t, J=7Hz), 1.20-1.43 (30H, m), 1.70 (2H, m), 2.30 (2H, m), 2.81 (2H, m), 3.80 (2H, t, J=7Hz), 4.39 (2H, q, J=17Hz), 6.63-8.26 (13H, m). |
| 83 | 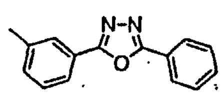 | $-CH_2COOH$ | 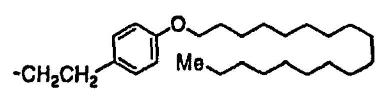 | 0.88 (3H, t, J=7Hz), 1.20-1.43 (30H, m), 1.70 (2H, m), 2.46 (2H, t, J=8Hz), 2.89 (2H, t, J=8Hz), 3.85 (2H, t, J=7Hz), 4.48 (3H, s), 6.72-8.16 (13H, m). |

| Ex. No. | 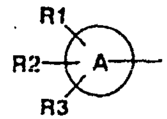 | 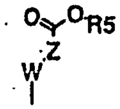 | —R4 | $^1$H NMR (300MHz, δ ppm in $CDCl_3$) |
|---|---|---|---|---|
| 84 | 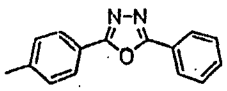 | $-CH_2COOH$ | 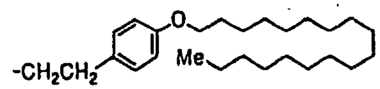 | 0.88 (3H, t, J=7Hz), 1.20-1.47 (30H, m), 1.73 (2H, m), 2.47 (2H, t, J=8Hz), 2.88 (2H, t, J=8Hz), 3.88 (2H, t, J=7Hz), 4.45 (2H, s), 6.72-8.16 (13H, m). |
| 85 | 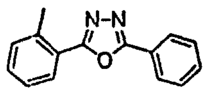 | Me<br>$-CHCOOH$ | 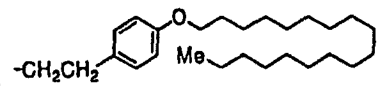 | 0.88 (3H, t, J=7Hz), 1.30 (3H, d, J=7Hz), 1.00-1.50 (30H, m), 1.69 (2H, m), 2.25 (2H, m), 2.82 (2H, m), 3.80 (2H, t, J=7Hz), 4.57 (1H, q, J=7Hz), 6.65-8.27 (13H, m). |
| 86 | 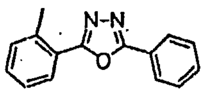 | $-CH_2CH_2CH_2COOH$ | 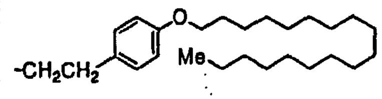 | 0.88 (3H, t, J=7Hz), 1.00-1.50 (30H, m), 1.60-1.94 (4H, m), 2.20 -2.52 (4H, m), 2.68-3.00 (2H, m), 3.13 (1H, dt, J=8,6Hz), 3.81(2H, t, J=7Hz), 4.32 (1H, dt, J=8,6Hz), 6.66-8.33 (13H, m). |
| 87 | 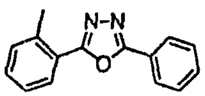 | $-CH_2COOH$ | 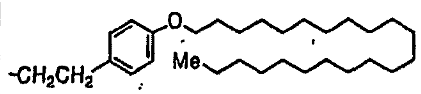 | 0.88 (3H, t, J=7Hz), 1.20-1.43 (38H, m), 1.69 (2H, m), 2.30 (2H, m), 2.81 (2H, m), 3.80 (2H, t, J=7Hz), 4.39 (2H, q, J=17Hz), 6.63-8.26 (13H, m). |
| 88 | 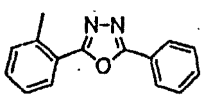 | $-CH_2COOH$ | 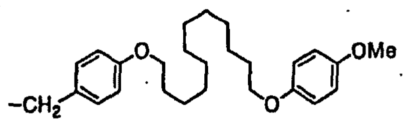 | 1.63-1.67 (20H, m), 1.76 (2H, m), 3.37 (1H, d, J=10Hz), 3.46 (1H, d, J=10Hz), 3.56 (4H, m), 3.78 (3H,s), 3.90 (H, t, J=7Hz), 4.09 (1H, d, J=17Hz), 4.75 (1H, d, J=17Hz), 6.51-8.16 (17H, m). |
| 89 | 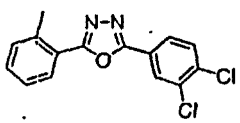 | $-CH_2COOH$ | 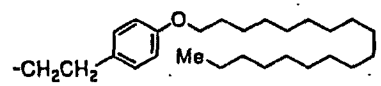 | 0.88 (3H, t, J=7Hz), 1.20-1.43 (30H, m), 1.69 (2H, m), 2.31 (2H, m), 2.82 (2H, t, J=8Hz), 3.78 (2H, t, J=7Hz), 4.38 (2H, q, J=17Hz), 6.60-8.26 (11H, m). |

| Ex. No. | 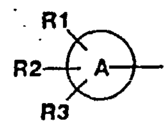 | 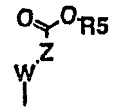 | —R4 | $^1$H NMR (300MHz, δ ppm in $CDCl_3$) |
|---|---|---|---|---|
| 90 | 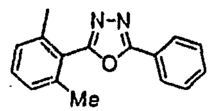 | $-CH_2COOH$ | 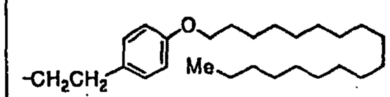 | 0.88 (3H, t, J=7Hz), 1.20-1.43 (30H, m), 1.73 (2H, m), 2.41 (2H, t, J=8Hz), 2.48 (3H, s), 2.83 (2H, m), 3.86 (2H, t, J=7Hz), 4.25 (2H, q, J=17Hz), 6.71-8.05 (12H, m). |
| 91 | 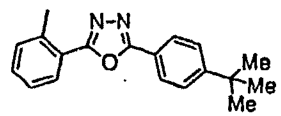 | $-CH_2COOH$ | 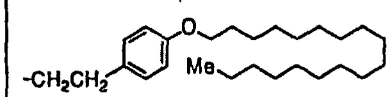 | 0.88 (3H, t, J=7Hz), 1.20-1.43 (30H, m), 1.69 (2H, m), 2.31 (2H, m), 2.82 (2H, m), 3.80 (2H, t, J=7Hz), 4.39 (2H, q, J=17Hz), 6.63-8.26 (12H, m). |
| 92 | 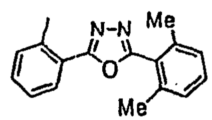 | $-CH_2COOH$ | 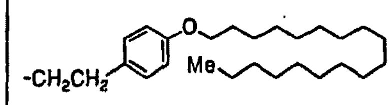 | 0.88 (3H, t, J=7Hz), 1.20-1.43 (30H, m), 1.73 (2H, m), 2.15-2.40 (2H, m), 2.31 (6H, s), 2.70-2.95 (2H, m), 3.86 (2H, t, J=7Hz), 4.40 (2H, q, J=17Hz), 6.70-8.10 (11H, m). |
| 93 | 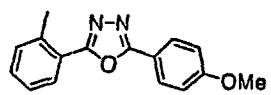 | $-CH_2COOH$ | 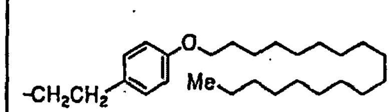 | 0.88 (3H, t, J=7Hz), 1.20-1.43 (30H, m), 1.70 (2H, m), 2.31 (2H, m), 2.82 (2H, m), 3.80 (2H, t, J=7Hz), 3.90 (3H, s), 4.38 (2H, q, J=17Hz), 6.63-8.25 (12H, m). |
| 94 | 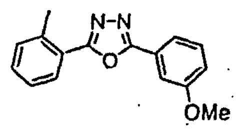 | $-CH_2COOH$ | 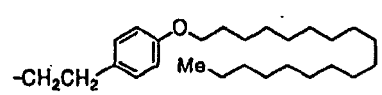 | 0.88 (3H, t, J=7Hz), 1.20-1.43 (30H, m), 1.70 (2H, m), 2.31 (2H, m), 2.82 (2H, m), 3.79 (2H, t, J=7Hz), 3.92 (3H, s), 4.39 (2H, q, J=17Hz), 6.62-8.28 (12H, m). |
| 95 | 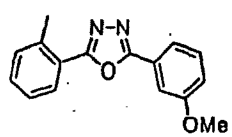 | 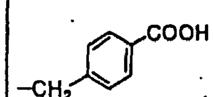 | 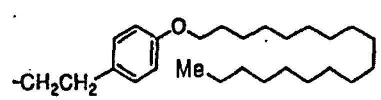 | 0.88 (3H, t, J=7Hz), 1.20-1.43 (30H, m), 1.72 (2H, m), 2.37 (2H, t, J=8Hz), 2.90 (2H, m), 3.83 (2H, t, J=7Hz), 3.94 (3H, s), 4.90 (2H, q, J=15Hz), 6.65-8.27 (16H, m). |

| Ex. No. | 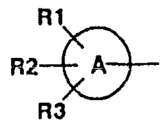 | 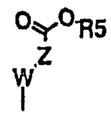 | —R4 | $^1$H NMR (300MHz, δ ppm in $CDCl_3$) |
|---|---|---|---|---|
| 96 | 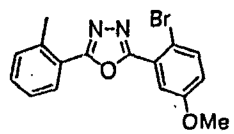 | $-CH_2COOH$ | 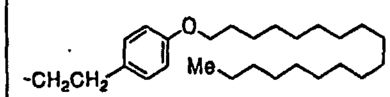 | 0.88 (3H, t, J=7Hz), 1.20-1.43 (30H, m), 1.70 (2H, m), 2.31 (2H, m), 2.82 (2H, m), 3.82 (2H, t, J=7Hz), 3.89 (3H, s), 4.40 (2H, q, J=17Hz), 6.66-8.24 (11H, m). |
| 97 | 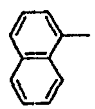 | $-CH_2COOH$ | 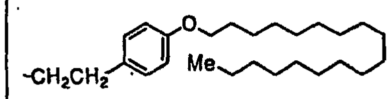 | 0.88 (3H, t, J=7Hz), 1.20-1.48 (30H, m), 1.73 (2H, m), 2.16-2.45 (2H, m), 2.84 (2H, m), 3.87 (2H, t, J=7Hz), 4.45 (2H, q, J=17Hz), 6.68-7.93 (11H, m). |
| 98 | 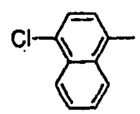 | $-CH_2COOH$ | 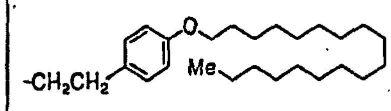 | 0.88 (3H, t, J=7Hz), 1.20-1.43 (30H, m), 1.74 (2H, m), 2.13-2.43 (2H, m), 2.84 (2H, m), 3.88 (2H, t, J=7Hz), 4.42 (2H, q, J=17Hz), 6.69-8.35 (10H, m). |
| 99 | 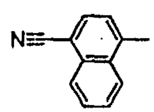 | $-CH_2COOH$ | 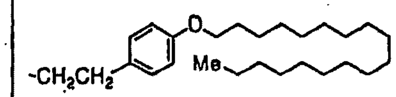 | 0.88 (3H, t, J=7Hz), 1.20-1.43 (30H, m), 1.74 (2H, m), 2.13-2.40 (2H, m), 2.83 (2H, m), 3.88 (2H, t, J=7Hz), 4.42 (2H, q, J=17Hz), 6.69-8.33 (10H, m). |
| 100 | 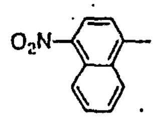 | $-CH_2COOH$ | 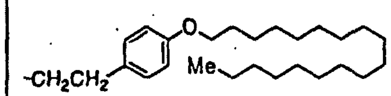 | 0.88 (3H, t, J=7Hz), 1.20-1.43 (30H, m), 1.74 (2H, m), 2.16-2.40 (2H, m), 2.85 (2H, m), 3.88 (2H, t, J=7Hz), 4.42 (2H, q, J=17Hz), 6.69-8.56 (10H, m). |
| 101 | 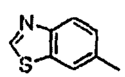 | $-CH_2COOH$ | 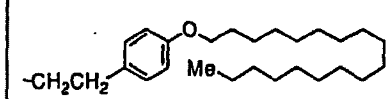 | 0.88 (3H, t, J=7Hz), 1.20-1.43 (30H, m), 1.75 (2H, m), 2.41 (2H, t, J=8Hz), 2.87 (2H, t, J=8Hz), 3.90 (2H, t, J=7Hz), 4.44 (2H, s), 6.75-8.14 (7H, m), 9.10 (1H, s). |

| Ex. No. | 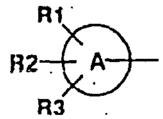 | 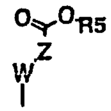 | —R4 | $^1$H NMR (300MHz, δ ppm in $CDCl_3$) |
|---|---|---|---|---|
| 102 | 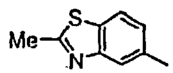 | $-CH_2COOH$ | 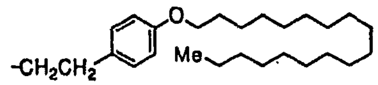 | 0.88 (3H, t, J=7Hz), 1.20-1.43 (30H, m), 1.72 (2H, m), 2.42 (2H, m), 2.86 (2H, t, J=8Hz), 2.89 (3H, s), 3.86 (2H, t, J=7Hz), 4.45 (2H, broad s), 6.71-8.16 (7H, m). |
| 103 | 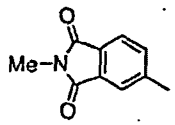 | $-CH_2COOH$ | 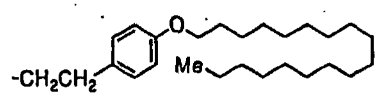 | 0.88 (3H, t, J=7Hz), 1.20-1.43 (30H, m), 1.75 (2H, m), 2.41 (2H, t, J=7Hz), 2.87 (2H, t, J=8Hz), 3.19 (3H, s), 3.90 (2H, t, J=7Hz), 4.42 (2H, s), 6.75-7.85 (7H, m). |
| 104 | 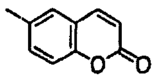 | $-CH_2COOH$ | 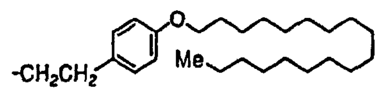 | 0.88 (3H, t, J=7Hz), 1.20-1.43 (30H, m), 1.76 (2H, m), 2.38 (2H, m), 2.87 (2H, m), 3.91 (2H, t, J=7Hz), 4.39 (2H, d, J=10Hz), 6.46-7.59 (9H, m). |
| 105 | 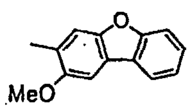 | $-CH_2COOH$ | 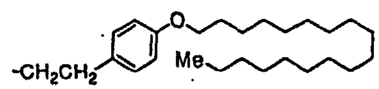 | 0.88 (3H, t, J=7Hz), 1.20-1.43 (30H, m), 1.74 (2H, m), 2.40 (2H, m), 2.86 (2H, m), 3.87 (2H, t, J=7Hz), 3.92 (3H, s), 4.41 (2H, q, J=17Hz), 6.71-7.93 (10H, m). |
| 106 | 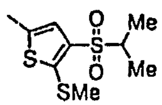 | $-CH_2COOH$ | 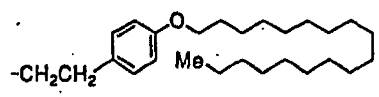 | 0.88 (3H, t, J=7Hz), 1.20-1.43 (36H, m), 1.75 (2H, m), 2.30-2.52 (2H, m), 2.58 (3H, s), 2.90 (2H, t, J=8Hz), 3.04 (1H, m), 3.90 (2H, t, J=7Hz), 4.37 (2H, q, J=18Hz), 6.76-7.10 (5H, m). |
| 107 | 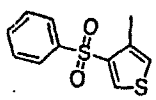 | $-CH_2COOH$ | 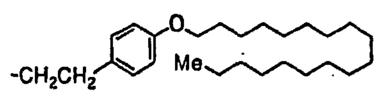 | 0.88 (3H, t, J=7Hz), 1.20-1.43 (31H, m), 1.74 (2H, m), 1.95 (1H, m), 2.35 (1H, m), 2.72 (1H, m), 3.88 (2H, t, J=7Hz), 4.20 (2H, q, J=18Hz), 6.71-8.30 (6H, m). |

| Ex. No. | 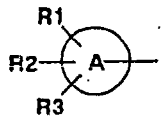 | 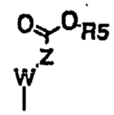 | —R4 | $^1$H NMR (300MHz, δ ppm in $CDCl_3$) |
|---|---|---|---|---|
| 108 | 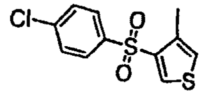 | $-CH_2COOH$ | 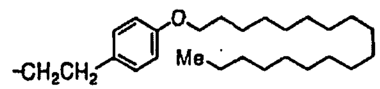 | 0.88 (3H, t, J=7Hz), 1.20-1.43 (30H, m), 1.74 (2H, m), 1.89-2.81 (4H, m), 3.89 (2H, t, J=7Hz), 4.28 (2H, q, J=18Hz), 6.73-8.29 (10H, m). |
| 109 | 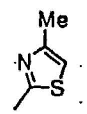 | $-CH_2COOH$ | 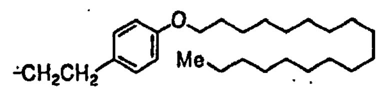 | 0.88 (3H, t, J=7Hz), 1.20-1.43 (30H, m), 1.76 (2H, m), 2.34 (3H, s), 2.82-3.04 (4H, m), 3.92 (2H, t, J=7Hz), 4.82 (2H, broad s), 6.59-7.14 (5H, m). |
| 110 | 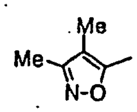 | $-CH_2COOH$ | 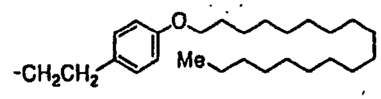 | 0.88 (3H, t, J=7Hz), 1.20-1.43 (30H, m), 1.75 (2H, m), 1.87 (3H, s), 2.22 (3H, s), 2.44 (2H, t, J=8Hz), 2.88 (2H, t, J=8Hz), 3.90 (2H, t, J=7Hz), 4.33 (2H, s), 6.77-7.04 (4H, m). |
| 111 | 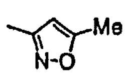 | $-CH_2COOH$ | 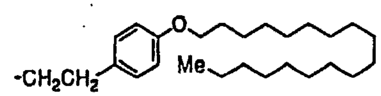 | 0.88 (3H, t, J=7Hz), 1.20-1.43 (30H, m), 1.76 (2H, m), 2.41 (3H, s), 2.67-2.93 (4H, m), 3.91 (2H, t, J=7Hz), 4.50 (2H, broad s), 6.79-7.12 (5H, m). |
| 112 | 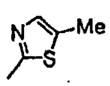 | $-CH_2COOH$ | 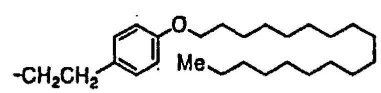 | 0.88 (3H, t, J=7Hz), 1.20-1.43 (30H, m), 1.76 (2H, m), 2.39 (3H, s), 2.83-3.01 (4H, m), 3.92 (2H, t, J=7Hz), 4.78 (2H, broad s), 6.78-7.14 (5H, m). |
| 113 | 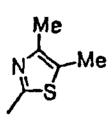 | $-CH_2COOH$ | 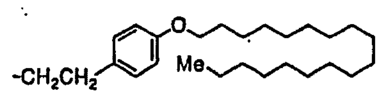 | 0.88 (3H, t, J=7Hz), 1.20-1.43 (30H, m), 1.76 (2H, m), 2.23 (3H, s), 2.28 (3H, s), 2.78-3.05 (4H, m), 3.92 (2H, t, J=7Hz), 4.73 (2H, broad s), 6.81-7.14 (4H, m). |

Example 114:

Preparation of N-[3-{4-(1-dodecyloxy)phenyl}propionyl]-N-(2-phenoxyphenyl) glycine methyl ester

A suspension of 3-{4-(1-dodecyloxy)phenyl}propionic acid (0.60 g, 1.79 mmol) and thionyl chloride (0.64 g, 5.38 mmol) in benzene (7 ml) was refluxed under heating for 0.5 hours. To a residue obtained by evaporating the solvent under reduced pressure was added dichloromethane (14 ml) and, after adding thereto successively 2-phenoxyaniline (0.30 g, 1.63mmol) and triethylamine (0.54 g, 5.38 mmol) , the mixture was stirred at room temperature for 5 hours. The solvent was evaporated from the reaction solution, and the resultant residue was dissolved in ethyl acetate, washed with successive, water and a saturated sodium chloride aqueous solution, and dried over anhydrous magnesium sulfate, followed by evaporating the solvent under reduced pressure. Thus, there was obtained a crude crystal (0.65 g, 79%) of 3-[4-(1-dodecyloxy)phenyl]-N-(2-phenoxyphenyl)propionamide. A solution of 0.30 g (0.60 mmol) of the thus-obtained crude crystal in N,N-dimethylformamide (4 ml) was added to 60% sodium hydride (0.036 g, 0.70 mmol) having been washed1 with n-hexane and, after stirring at room temperature for 20 minutes, methyl bromoacetate (0.14 g, 0.90 mmol) was added thereto, followedby stirring at room temperature for 48 hours. The reaction solution was diluted with ethyl acetate, washed with successive, water and a saturated sodium chloride aqueous solution, and dried over anhydrous magnesium sulfate, followed by evaporating the solvent under reduced pressure. Purification of the thus-obtained residue through silica gel column chromatography (eluent: n-hexane/ethyl acetate = 5:1) yielded N-[3-{4-(1-dodecyloxy)phenyl}propionyl]-N-(2-phenoxyphenyl)glycine methyl ester (0.23 g) as a colorless oil.
$^1$H-NMR (300MHz, $\delta$ ppm in $CDCl_3$)
0.88 (3H, t, J=7Hz), 1.20-1.45 (20H, m), 1.74 (2H, m), 2.43 (2H, m), 2.83 (2H,m), 3.69 (1H, d, J=17Hz), 3.73 (3H, s) , 3.89 (2H, t, J=7Hz), 4.87 (1H, d, J=17Hz), 6.72-7.50 (13H, m)

Examples 115 to 121:

The following compounds of the present invention were obtained according to the process for producing the compound of Example 114.
N-[2-(2-Methylphenoxy)phenyl]-N-[3-{-4-(1-octadecyloxy)phenyl}propionyl]glycine t-butyl ester (Example 115)
$^1$H-NMR (300MHz, $\delta$ ppm in $CDCl_3$) :
0.88 (3H, t, J=7Hz), 1.20-1.43 (30H, m), 1.74 (2H, m), 2.19 (3H, s) , 2.47 (2H, m) , 2.87 (2H, m) , 3.88 (2H, t, J=7Hz), 4.23 (2H, q, J=17Hz), 6.70-7.52 (12H, m)
N-[2-(5-Phenyl-1,3,4-oxadiazol-2-yl)phenyl]-N-[3-{4-(1-octadecyloxy)phenyl}propionyl]glycine t-butyl ester (Example 116)
$^1$H-NMR (300MHz, $\delta$ ppm in $CDCl_3$) :
0.88 (3H, t, J=7Hz), 1.20-1.40 (30H, m), 1.45 (9H, s), 1.67 (2H, m), 2.35 (2H, m), 2.83 (2H, m), 3.50 (1H, d, J=17Hz), 3.75 (2H, t, J=7Hz), 5.01 (1H, d, J=17Hz), 6.59(2H, m), 6.88 (2H, m), 7.48-7.62 (5H, m), 7.74 (1H, m), 8.00-8.08 (2H, m), 8.33 (1H, m)
N-[4-{12-(4-Methoxyphenoxy)dodecyloxyl}phenylacetyl]-N-[2-(5-phenyl-1,3,4-oxadiazol-2-yl)phenyl]glycine t-butyl ester (Example 117)
$^1$H-NMR (300MHz, $\delta$ ppm in $CDCl_3$):
1.20-1.65 (20H, m), 1.45 (9H, s), 1.76 (2H, m), 3.24 (1H, m), 3.55 (1H, d, J=17Hz), 3.62 (1H, m), 3.77 (3H,s), 3.90 (2H, t, J=7Hz), 5.05 (1H, d, J=17Hz), 6.50 (2H, m) , 6.75 (2H, m), 6.83 (4H, m), 7.48-7.68 (3H, m), 7.80 (1H, m), 7.95-8.03 (2H, m), 8.25 (1H, m)
t-Butyl 2-amino-N-[2-(5-phenyl-1,3,4-oxadiazol-2-yl)phenyl]-N-[3-{4-(1-octadecylocy)phenyl}propionyl propionate (Example 118)
$^1$H-NMR (300MHz, $\delta$ ppm in $CDCl_3$):
0.88 (3H, t, J=7Hz), 0.94 (3H, d, J=7Hz), 1.20-1.40 (30H, m), 1.51 (9H, s), 1.68 (2H, m), 2.26 (2H, m), 2.80 (2H, m), 3.75 (2H, t, J=7Hz), 5.00 (1H, q, J=7Hz), 6.60 (2H, m), 6.85 (2H, m), 7.48-7.68 (5H, m), 7.83 (1H, m), 8.00-8.08 (2H, m), 8.33 (1H, m)
Ethyl 4-amino-N-[2-(5-phenyl-1,3,4-oxadiazol-2-yl)phenyl]-N-[3-{4-(1-octadecylocy)phenyl}propionyl]butanoate (Example 119)
$^1$H-NMR (300MHz, $\delta$ ppm in $CDCl_3$):
0.88 (3H, t, J=7Hz), 1.19 (3H, t, J=7Hz), 1.00-1.46 (30H, m), 1.63-1.96 (4H, m), 2.28 (4H, m), 2.80 (2H, m), 3.04 (1H, m), 3.80 (2H, t, J=7Hz), 4.06 (2H, q, J=7Hz), 4.30 (1H, m), 6.65 (2H, m), 6.90 (2H, m), 7.08 (1H, m), 7.56 (5H, m) , 8.08 (2H,m), 8.34 (1H, m)
N-[3-{4-(1-Octadecyloxy)phenyl}propionyl]-N-(2,4,6-trimethylphenyl)glycine methyl ester (Example 120)
$^1$H-NMR (300MHz, $\delta$ ppm in $CDCl_3$):

0.88 (3H, t, J=7Hz), 1.17-1.48 (30H, m), 1.74 (2H, m), 2.15 (3H, s), 2.23 (2H, m), 2.27 (3H, s), 2.85 (2H, m) , 3.76 (3H, s), 3.89 (2H, t, J=7Hz), 4.09 (2H, s), 6.76 (2H, m), 6.89 (2H, s), 6.99 (2H, m)
N-(2-Phenyl)-N-[3-{4-(1-octadecyloxy)phenyl}propionyl]glycine t-butyl ester (Example 121)
$^1$H-NMR (300MHz, $\delta$ ppm in $CDCl_3$):
0.88 (3H, t, J=7Hz), 1.20-1.46 (39H, m), 1.78-1.69 (2H, m), 2.38 (2H, t, J=8Hz), 2.85 (2H, t, J=8Hz), 3.89 (2H, t, J=7Hz), 4.23 (2H, s), 6.75-7.22 (9H, m)

Test Example 1 [VEGF receptor-binding test]:

According to the method described in a document (Cell Growth & Differentiation, vol. 7, pp. 213-221, 1996), the following tests were carried out.

NIH3T3 cells, in which Flt-1 was forced to be expressed, were seeded on a 24-well collagen-coated plate ($7x10^4$ cells/well), and were cultured in Dulbecco' s modified Eagle' s medium (DMEM) containing 10% bovine serum and 200 µg/ml of Geneticin G418 for 24 hours at 37°C under an atmosphere of 5% carbon dioxide gas. The cells were preincubated in Buffer A [containing 10 mM HEPES (N-2-hydroxyethylpiperazine-N'-2-ethanesulfonic acid) and 0.1% BSA (bovine serum albumin) in DMEM] for 30 minutes at 4°C. Subsequently, the medium was replaced with Buffer B (containing 10 mM HEPES and 0.5% BSA in DMEM). A test solution prepared by dissolving each of the test compounds shown in Table 2 in dimethylsulfoxide and subsequently diluting the solution into a prescribed concentration with Buffer B, and [$^{125}$I]-VEGF (the final concentration was set to 25 pM) were added thereto. Binding reaction was carried out for 90 minutes at 4°C. After completion of the reaction, the cells were washed three times with ice-cooled Buffer A. Subsequently, 0.5 ml of 0.5 M NaOH was added to each well, and the cells were lysed in 30 minutes at room temperature. The radioactivity of the lysed cells in each well was measured by means of a gamma counter, and a total binding quantity of [$^{125}$I]-VEGF was calculated. Non-specific binding quantity of [$^{125}$I]-VEGF was measured by competition assay in the presence of 10 nM non-labeled VEGF. The specific binding quantity of [$^{125}$I]-VEGF was calculated from the difference between the total binding quantity of [$^{125}$I]-VEGF and non-specific binding quantity of [$^{125}$I]-VEGF. The binding inhibition index of the test compounds was calculated by the following equation.

Binding inhibition index (%) =

$$\left(1 - \frac{\text{Specific binding quantity of } [^{125}\text{I}]\text{-VEGF of the group with an addition of test compounds}}{\text{Specific binding quantity of } [^{125}\text{I}]\text{-VEGF of the control group}}\right) \times 100$$

Table 1

| Test Compound (Example No.) | Inhibition Index (%) | Concentration (pg/ml) |
|---|---|---|
| 36 | 40 | 3 |
| 57 | 61 | 1 |
| 82 | 59 | 1 |
| 91 | 55 | 3 |
| 94 | 53 | 3 |
| 95 | 55 | 3 |
| 96 | 60 | 3 |
| a | 65 | 1 |

a: 5-Amino-2-(4-carboxyphenoxy)-N-[3-{4-(1-octadecyloxy)phenyl}propionyl]benzoic acid; an aminobenzoic acid derivative described in JP-A-12-072653 (WO01/02344)

Test Example 2 [Solubility test]:

1 mg of a test compound was weighed out in a threaded testtube, 1 ml of a phosphate buffer (pH: 6.8; 20mM; ionintensity = 0.15) was added thereto and, after shaking for 2 hours at room temperature under light-shielded condition, was incubated for 22 hours at 25°C. A supernatant obtained by centrifuging the test tube for 10 minutes at 3000 rpm at 25°C was further centrifuged for 10 minutes at 11000 rpm at 25°C. The thus-obtained supernatant was diluted with a water/acetonitrile (1:1) mixture with a dilution ratio of 2 times to 21 times to prepare a sample solution (A). Also, 0.5

mg of the test compound was dissolved in 5 ml of acetonitrile, and diluted two times with acetonitrile to prepare a standard solution (B). 10 µl of the sample solution (A) and 10 µl of the standard solution (B) were subjected to measurement according to HPLC method to determine a peak area of the test compound in each of the solutions. The solubility (mg/ml) of the test compound was calculated according to the following equation using the measured values.

Solubility (mg/ml) of the test compound =

$$\text{Concentration of test compound (mg/ml) in (B)} \times \frac{\text{Peak area of test compound in (A)}}{\text{Peak area of test compound in (B)}} \times \text{Dilution ratio}$$

<HPLC-operating conditions>

Column: shiseido capcell pak UG120 (4.6x150mm)
Mobile phase: acetonitrile/10 mM ammonium acetate mixture (90:10)
Detecting wavelength: 230 nm
Flow rate: 1 ml/min
Column temperature: 40 °C

Table 2

| Test Compound (Example No.) | pH | Solubility (µg/ml) |
|---|---|---|
| 82 | 6.7 | 353.5 |
| 91 | 6.8 | 168.2 |
| 94 | 6.9 | 216.1 |
| 96 | 6.7 | 381.9 |
| a | 6.9 | 0.029 |

Industrial Applicability

The carboxylic acid compounds of the invention represented by the formula (1) or the pharmaceutically acceptable salts thereof have a VEGF receptor antagonistic action and are useful for treating diseases involving VEGF. The compounds of the present invention inhibit VEGF-dependent proliferation of vascular endothelial cells and inhibit arterialization by inhibiting a ligand (VEGF) from binding to a VEGF receptor. The compounds also inhibit vascular hyperpermeability due to VEGF. Here, examples of diseases and pathologic symptoms, in which VEGF is involved, includediabatic retinopathy and other retinopathy, chronic rheumatism, solid tumors, cerebral edema and damages relating to ischemia reperfusion injury, psoriasis, atherosclerosis, retrolental fibroplasia, angiogenic glaucoma, macular degeneration due to aging, thyroid gland hyperplasia (including Graves' disease), chronic inflammation, pneumonia, nephritic syndrome, paraneoplastic hypofunction of immunity, ascites retention, endocardium exudation (relating to endocarditis, etc.) and hydrothorax retention.

## Claims

**1.** A carboxylic acid derivative represented by formula:

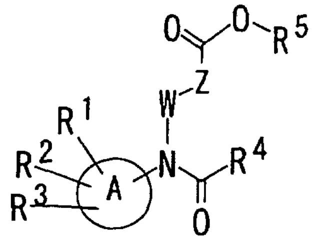

wherein ring A represents a benzene ring, a naphthalene ring or a hetero ring containing 1 to 4 hetero atoms arbitrarily selected from among a nitrogen atom, an oxygen atom and a sulfur atom,
W represents a $C_{1-5}$ alkylene group,
Z represents a single bond or a phenylene group,
$R^1$ and $R^2$ are the same or different and each represents a hydrogen atom, a halogen atom, a $C_{1-5}$ alkyl group or a $C_{1-10}$ alkoxy group,
$R^3$ represents a hydrogen atom, a halogen atom, a $c_{1-12}$ alkyl group, a $C_{2-5}$ alkynyl group, a trifuloromethyl group, an acetynyl group, a cyano group, a nitro group, a group represented by $-CH_2-R^6$ [wherein $R^6$ represents a $C_{1-5}$ alkylthio group, a group represented by formula:

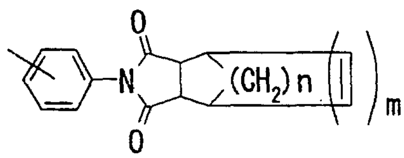

(wherein m represents 0 or 1, and n represents an integer of from 0 to 3)], a group represented by formula:

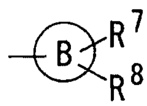

[wherein ring B represents a monocyclic hetero ring containing 1 to 3 hetero atoms arbitrarily selected from among a nitrogen atom, an oxygen atom and a sulfur atom, or a benzene ring, $R^7$ represents a hydrogen atom or a $C_{1-5}$ alkyl group, $R^8$ represents a hydrogen atom or a $C_{1-5}$ alkyl group or a group represented by formula:

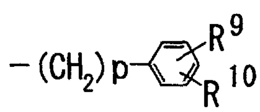

(wherein $R^9$ and $R^{10}$ are the same or different and each represents a hydrogen atom, a halogen atom, a $C_{1-5}$ alkyl group or a $C_{1-5}$ alkoxy group, and p represents an integer of from 0 to 8)], or a group represented by $-Y-R^{11}$ (wherein Y represents a group represented by -CO-, -O-, -S- or $-SO_2-$, and $R^{11}$ represents a $C_{1-10}$ alkyl group, a methyl group substituted by 1 to 3 fluorine atoms, a phenyl group, a phenyl group substituted by a $C_{1-5}$ alkyl group, a phenyl group substituted by a $C_{1-5}$ alkoxy group, a $C_{2-8}$ dialkylamino group or a cyclic amino group)],
$R^4$ represents a group represented by formula:

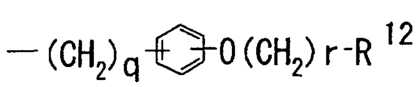

(wherein $R^{12}$ represents a hydrogen atom or a phenyloxy group substituted by a $C_{1-5}$ alkoxy group, q represents an integer of from 1 to 5, and r represents an integer of from 10 to 24), and
$R^5$ represents a hydrogen atom or a $C_{1-5}$ alkyl group, or a pharmaceutically acceptable salt of the derivative.

**2.** The carboxylic acid derivative or the pharmaceutically acceptable salt of the derivative according to Claim 1, wherein the ring A is a benzene ring, a naphthalene ring, a thiophene ring, a thiazole ring, an isoxazole ring, a benzothiazole ring, a phthalimide ring, a coumarin ring or a dibenzofuran ring, and the ring B is a benzene ring, an oxazole ring or an oxadiazole ring.

**3.** The carboxylic acid derivative or the pharmaceutically acceptable salt of the derivative according to Claim 2, wherein m is 1, n is an integer of from 1 to 3 and; when Y represents -CO-, -O- or -S-, $R^{11}$ is a $C_{1-10}$ alkyl group, a methyl group substituted by 1 to 3 fluorine atoms, a phenyl group, a phenyl group substituted by a $C_{1-5}$ alkyl group, or a phenyl group substituted by a $C_{1-5}$ alkoxy group and; when Y represents $-SO_2-$, $R^{11}$ represents a $C_{2-8}$ dialkylamino group or a cyclic amino group.

**4.** The carboxylic acid derivative or the pharmaceutically acceptable salt of the derivative according to Claim 3, wherein the ring A is a benzene ring, and $R^1$ and $R^2$ are the same or different and each represents a hydrogen atom or a $C_{1-5}$ alkyl group, $R^3$ is a group represented by formula:

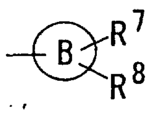

or a group represented by $-Y-R^{11}$, the ring B is an oxazole ring or an oxadiazole ring, Y is -CO- or O-, and $R^{11}$ is a phenyl group, a phenyl group substituted by a $C_{1-5}$ alkyl group or a phenyl group substituted by a $C_{1-5}$ alkoxy group.

**5.** The carboxylic acid derivative or the pharmaceutically acceptable salt of the derivative according to Claim 4, wherein $R^3$ is a group represented by formula:

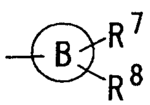

and the ring B is an oxazole ring or an oxadiazole ring.

**INTERNATIONAL SEARCH REPORT**

International application No.
PCT/JP02/13692

A. CLASSIFICATION OF SUBJECT MATTER

Int.Cl[7] C07C235/38, 255/60, 311/29, 317/32, 323/41, C07D209/48, 209/76, 261/14, 263/32, 271/10, 277/28, 277/42, C07D277/68, 295/22, 307/91, 311/14, 333/34//A61K31/198, 31/343, 31/352,

According to International Patent Classification (IPC) or to both national classification and IPC

B. FIELDS SEARCHED

Minimum documentation searched (classification system followed by classification symbols)

Int.Cl[7] C07C, C07D, A61K, A61P

Documentation searched other than minimum documentation to the extent that such documents are included in the fields searched

Electronic data base consulted during the international search (name of data base and, where practicable, search terms used)

CAPLUS(STN), REGISTRY(STN), CAOLD(STN)

C. DOCUMENTS CONSIDERED TO BE RELEVANT

| Category* | Citation of document, with indication, where appropriate, of the relevant passages | Relevant to claim No. |
|---|---|---|
| X | EP 262399 A2 (Takeda Chemical Industries, Ltd.), 06 April, 1988 (06.04.88), Claim 21; page 17, compound 3-b & JP 63-159367 A & US 4925868 A & DE 3783988 G & CA 1321390 C | 1 |
| A | WO 01/02344 A1 (Taisho Pharmaceutical Co., Ltd.), 11 January, 2001 (11.01.01), Claims; page 42, example 19 & JP 2001-72653 A & AU 200055726 A & EP 1193246 A1 & KR 2002013942 A | 1-5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 March, 2003 (25.03.03) | 08 April, 2003 (08.04.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13692

Continuation of A. CLASSIFICATION OF SUBJECT MATTER
(International Patent Classification (IPC))

Int.Cl[7] 31/381, 31/40, 31/403, 31/4035, 31/42, A61K31/421, 31/4245, 31/426, 31/428, 31/4453, 34/5375, A61P3/00, 9/00, 27/02, 29/00, 35/00, 43/00

(According to International Patent Classification (IPC) or to both national classification and IPC)

Form PCT/ISA/210 (extra sheet) (July 1998)